(19) **Europäisches Patentamt**
**European Patent Office**
**Office européen des brevets**

(11) **EP 2 795 496 B1**

(12) **EUROPÄISCHE PATENTSCHRIFT**

(45) Veröffentlichungstag und Bekanntmachung des Hinweises auf die Patenterteilung:
**05.08.2020  Patentblatt 2020/32**

(51) Int Cl.:
*G16H 40/63* (2018.01)    *G16H 10/60* (2018.01)

(21) Anmeldenummer: **12824911.7**

(22) Anmeldetag: **20.12.2012**

(86) Internationale Anmeldenummer:
**PCT/IB2012/057559**

(87) Internationale Veröffentlichungsnummer:
**WO 2013/093852 (27.06.2013 Gazette 2013/26)**

(54) **NAVIGIERBARE DARSTELLUNG EINER LÖSUNGSVIELFALT VON THERAPIEPLÄNEN**

NAVIGABLE PRESENTATION OF A VARIETY OF SOLUTIONS FOR THERAPY PLANS

REPRÉSENTATION GRAPHIQUE NAVIGABLE D'UNE PLURALITÉ DE SOLUTIONS DE PROGRAMMES THÉRAPEUTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2011  DE 102011057038**

(43) Veröffentlichungstag der Anmeldung:
**29.10.2014  Patentblatt 2014/44**

(73) Patentinhaber: **Fraunhofer Gesellschaft zur Förderung der angewandten Forschung e.V.**
**80686 München (DE)**

(72) Erfinder:
- **SCHERRER, Alexander**
  **67663 Kaiserslautern (DE)**
- **KUEFER, Karl-Heinz**
  **67685 Weilerbach (DE)**
- **SUESS, Philipp**
  **55257 Budenheim (DE)**
- **BORTZ, Michael**
  **67663 Kaiserslautern (DE)**

(74) Vertreter: **Leonhard, Frank Reimund et al**
**Leonhard & Partner**
**Patentanwälte**
**Postfach 10 09 62**
**80083 München (DE)**

(56) Entgegenhaltungen:
**WO-A2-2004/093971    WO-A2-2011/153639
US-A- 6 038 283    US-A1- 2005 111 621**

- **THIEKE ET AL: "A new concept for interactive radiotherapy planning with multicriteria optimization: First clinical evaluation", RADIOTHERAPY AND ONCOLOGY, ELSEVIER, IRELAND, Bd. 85, Nr. 2, 1. November 2007 (2007-11-01), Seiten 292-298, XP022363696, ISSN: 0167-8140, DOI: 10.1016/J.RADONC.2007.06.020**
- **BORTFELD ET AL: "Optimized planning using physical objectives and constraints", SEMINARS IN RADIATION ONCOLOGY, SAUNDERS, PHILADELPHIA, PA, US, Bd. 9, Nr. 1, 1. Januar 1999 (1999-01-01), Seiten 20-34, XP005454727, ISSN: 1053-4296, DOI: 10.1016/S1053-4296(99)80052-6**
- **MONZ M ET AL: "Pareto navigationâ algorithmic foundation of interactive multi-criteria IMRT planning; Pareto navigation", PHYSICS IN MEDICINE AND BIOLOGY, INSTITUTE OF PHYSICS PUBLISHING, BRISTOL GB, Bd. 53, Nr. 4, 21. Februar 2008 (2008-02-21), Seiten 985-998, XP020127372, ISSN: 0031-9155**
- **"Better Approach for Presenting Dose Volume Histogram (DVH)", IP.COM JOURNAL, IP.COM INC., WEST HENRIETTA, NY, US, 24. September 2008 (2008-09-24), XP013126132, ISSN: 1533-0001**

## Beschreibung

Die Erfindung befasst sich mit einer besonderen Art der grafischen Darstellung einer Lösungsvielfalt bei der klinischen, intensitätsmodulierten Strahlentherapie (IMRT). Letztere ist geprägt durch eine neue Art der mehrkriteriellen Optimierung (MCO). Dadurch ergibt sich mit geringem Zeitaufwand höhere Planqualität.

Mit einem Plan ist medizinisch gemeint, dass ein Patient eine Therapie erhält. Der Plan ist definiert über eine Vielzahl von technischen Parametern, die sich durch Einstellungen an dem Bestrahlungsgerät festlegen lassen. Es handelt sich um Zeiten, Intensitäten, Winkelpositionen, Längspositionen eines Behandlungstisches (je nach Therapiegerät), Art der Strahlung und einer Vielzahl anderer technischer Parameter, die für einen Patienten ermittelt werden müssen.

Man spricht - in der medizinischen Sprache gebräuchlich - von einem "Plan". Technisch gesprochen ist der Plan eine Vielzahl von technischen Parametern, die diesen Plan festlegen und die dem technischen Gerät vorgegeben werden. Das daraus folgende Therapieren oder Bestrahlen ist zwangsläufige Folge der vorhergehenden Einstellung der zutreffenden Parameter. Alle diese Parameter haben technische Eigenschaften, bilden also eine technische Größe im Gerät ab, die das Gerät, bei zutreffender Programmierung und zutreffender Funktionalität der Technik, auch realistisch umsetzt.

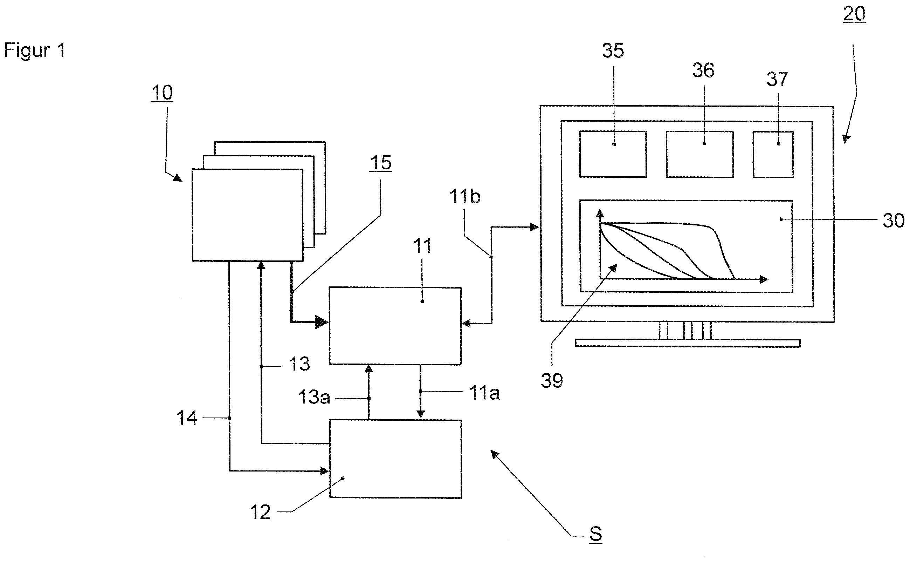

US 7,391,026 B2 zeigt ein solches Steuerverfahren oder Planungswerkzeug, welches die MCO mit der IMRT verbindet und eine Vielzahl von technischen Parametern für einen zu ermittelnden Plan definiert, die dem dort dargestellten Gerät aufgeschaltet werden können, vgl. dort Figur 1. Auf die dortige Beschreibung soll verwiesen werden.

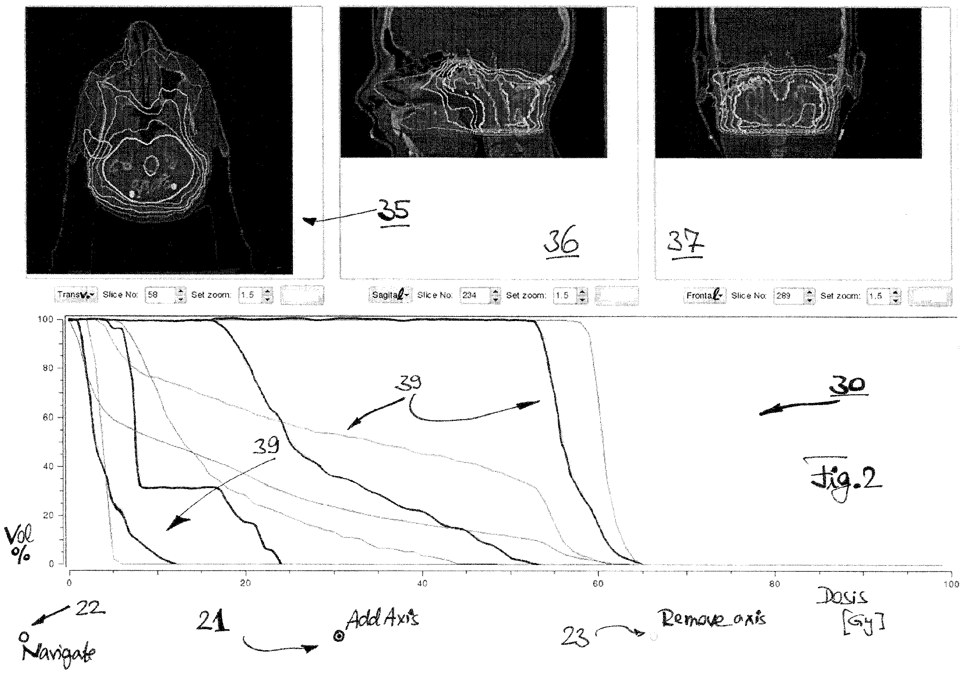

Aus der WO 2011/153639 A ist ein Verfahren für ein Strahlungs-Therapiegerät dem Fachmann zugänglich, das unter dem Begriff PARETO eine Optimierung der Strahlung nach Plänen vorschlägt. Es wird dazu insbesondere nach Figur 2 ein GUI zur Navigation beschrieben, das vier dargestellte Elemente enthält. Der SPOKE Plot links oben ist besonders wichtig und stellt die Kriterien des dargestellten Plans da. Das rechte obere Bild ist ein Dosis-Volumen-Histogramm, welches die aktuelle Therapieeinstellung repräsentiert. Ihre Eigenschaften sind im SPOKE Plot links dargestellt. Speziell auf Seite 24 werden die Funktionen und die Eigenschaften der GUI erläutert, aber ohne eine Möglichkeit der interaktiven (direkten) Änderung des DVH Diagramms.

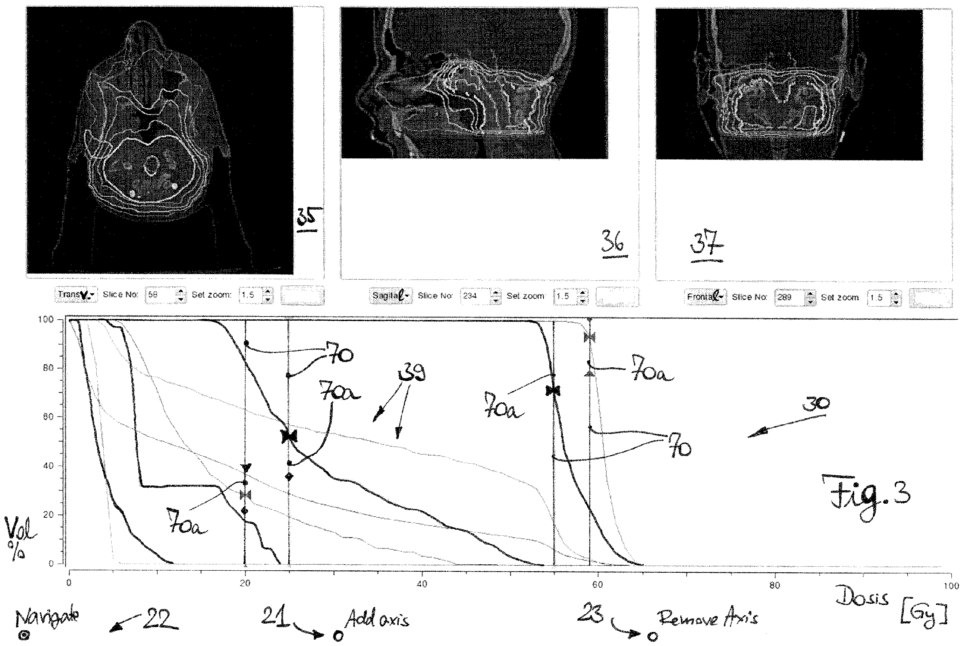

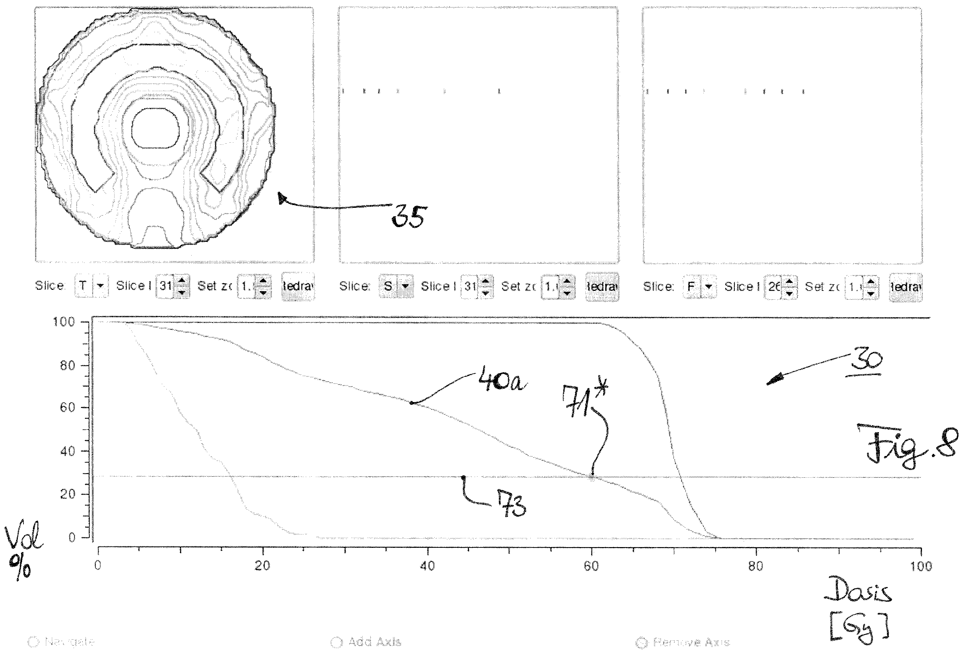

US 2005/0111621 A veranschaulicht eine Veränderung eines DVH-Diagramms an einer spezifischen Stelle, dort Figur 8, Bezugszeichen 196, wonach ein neuer Plan berechnet wird. Vergleichbar werden solche Verschiebefunktionen auch bei Isodosis-Darstellungen erklärt, dort Figuren 3 bis 6.

Ausgangspunkt der Erfindung und demgemäß **technische Aufgabe** ist es, dem Benutzer eine weitergehende, verfeinerte Variante an die Hand zu geben, die sich ein Stück von der Abstraktion löst, die US 7,391,026 B2 für die Navigation bereitstellt. Der Benutzer oder Planer soll es an die Hand bekommen, in seinen geübten und herkömmlichen Bahnen auch denken zu können und eine wichtige Orientierung in diesen geübten, herkömmlichen Bahnen bieten DVH-Kurvenscharen (Dosis Volumen Histogramme), die ihm (dem Benutzer/Planer) im Wege der Interaktion wesentliche Informationen für die Qualität eines 'Plans' liefern. In US 7,391,026 B2 hatte diese DVH-Kurvenschar nur eine darstellende Funktion, sie war (dort in Figur 6, links unten) eine Folge eines anderweitig festgelegten 'Plans'.

Die vorgenannte technische Aufgabe wird gemäß der beanspruchten Erfindung nach Anspruch 1 dadurch gelöst, dass das interaktive Auswahlverfahren mit den DVH-Kurvenscharen arbeitet, respektive interaktiv Lösungen aus der gespeicherten Vielfalt von Lösungen (entspricht den Plänen) herauszufinden.

Das interaktive Verfahren stellt eine Vielzahl von vorberechneten Lösungen optisch sichtbar dar. Dies erfolgt auf einer Darstellungseinrichtung, die einen gebräuchlichen Sichtschirm ebenso beinhalten kann, wie ein manuell bedienfähiges Tableau (Tablet PC). Die Vielzahl von vorberechneten Lösungen für mögliche Pläne, die vorberechnet in einer Datenbank gespeichert vorliegen, wird auf der Darstellungseinrichtung so dargestellt, dass es dem Benutzer ermöglicht wird, mit dieser Lösungsvielfalt zu arbeiten.

Die Steuerung der Darstellungseinrichtung erfolgt ausgehend von einer Basisdarstellung. Die Basisdarstellung liegt darin, dass ein DVH-Diagramm als Hauptdiagramm abgebildet wird. Auf diesem DVH-Diagramm ist eine Vielzahl von vorberechneten Lösungen darstellbar, es wird zu einem Zeitpunkt aber nur eine dieser Lösungen visuell dargestellt. Eine solche Lösung beinhaltet eine Gruppe von definierten Volumina, von denen jedem eine visuell dargestellte Kurve zugeordnet ist.

Die normale Betrachtungsweise einer DVH-Kurvendarstellung ist das Auftragen von Volumenprozenten über der Dosis. Ein Kurvenpunkt sagt aus, wie viel Volumenprozent einen Mindestbetrag an Strahlendosis erhalten. Ist ein Punkt ausgewählt, der beispielsweise eine Strahlendosis von 60 Gy (Gray) aufweist und auf der Volumenprozent-Achse einen entsprechenden Wert von 28% aufweist, sagt dies aus: Ein Dosiswert von zumindest 60 Gy wird in 28% des betrachteten Volumens erhalten (durch den Plan und die Therapie). Daraus versteht sich, dass eine Kurve, die ein Risiko repräsentiert, idealerweise sehr steil im Anfangsbereich (um 0 Gy bis 10 Gy) herum verläuft, also möglichst wenig Strahlungsdosis auf möglichst viel Volumen. Der umgekehrte Idealfall für ein Target (das betroffene Tumorvolumen) ist eine Kurve, die sehr lange bei 100% bleibt und erst sehr spät sehr steil abfällt, um eine möglichst hohe Prozentzahl des Volumens mit einem möglichst großen Dosiswert zu bestrahlen.

Dieses qualitative Wunsch-Ergebnis wird selten erreicht, meist liegen die Kurven zwischen diesen beiden Extrema. Risiken werden stärker bestrahlt, als an sich gewünscht, und Targets werden schwächer bestrahlt, als eigentlich gewünscht. Die Aufgabe liegt darin, Lösungen festzulegen, die mehrkriteriell möglichst so "optimiert" sind, dass die Risiken gerade noch erträglich bestrahlt werden und die Targets noch ausreichend Dosis erhalten, um den Tumor zu kontrollieren.

Die Darstellung einer Lösung als DVH-Diagramm basiert auf den betrachteten Volumina. Unter diesen Volumina sind Tumorbereich(e), gesunde Organe und anatomische Entitäten oder auch relevante und daher gesondert definierte Gewebebereiche (Volumen). Obwohl nur eine Lösung zu einer Zeit dargestellt ist, sind in der - hinter der Darstellung stehenden - Datenbank eine Vielzahl von anderen Lösungen verfügbar, die bereits vorberechnet sind und mehrkriteriell optimiert wurden. Sie werden im Wege der "Steuerung der Darstellung (Visualisierung)" auch interaktiv dargestellt, wie im Folgenden erläutert.

Neben dem Hauptdiagramm (Anspruch 1) kann zumindest ein Hilfsdiagramm vorgesehen sein (Anspruch 12), welches eine andere Darstellungsform abbildet, mit Blick auf die gerade in dem DVH-Diagramm als Hauptdiagramm abgebildete Lösung.

Das oder die Hilfsdiagramme können beispielsweise solche Diagramme sein, bei denen Isodosis-Linien (Isodosen) eingezeichnet sind, so drei Schnittdarstellungen in zueinander senkrechten Richtungen, bei denen zusätzlich die den DVH-Kurven im Hauptdiagramm entsprechenden Volumina durch geeignete Konturlinien oder Umrisslinien dargestellt werden. Jeder Kurve aus dem Hauptdiagramm wird so ein entsprechendes Volumen in den drei zueinander senkrechten Schnittdarstellungen zugeordnet, und zugehörig auch die Isodosis-Linien. Dies ist ein Beispiel von einer anderen Visualisierung (oder: Darstellung), außerhalb der Darstellung des DVH-Diagramms. Andere Möglichkeiten liegen darin, die drei Schnittdarstellungen durch eine dreidimensionale Darstellung zu ersetzen, so dass nur ein Hilfsdiagramm vorhanden ist. Diese dreidimensionale Darstellung kann am Lichtschirm auch rotiert werden, um aus unterschiedlichen Betrachtungsrichtungen dargestellt zu sein.

Die interaktive Auswahl erfasst passende Lösungen im Sinne von Therapieplänen, die sich jeweils aus einer Anzahl von technischen Steuerparametern zusammensetzen..

Ein Startpunkt wird auf einer der DVH-Kurven ausgewählt (Anspruch 1). Dieser Startpunkt liegt auf der ausgewählten DVH-Kurve, von denen mehrere im Hauptdiagramm dargestellt sind. Diese als "Kurvenschar" benannte Darstellung repräsentiert eine Lösung der Datenbank.

Auf einer Kurve dieser Kurvenschar ist oder wird ein Punkt als Startpunkt ausgewählt. Das System ordnet diesem Punkt eine gerade Achse zu, die durch den Startpunkt verläuft. Die gerade Achse schneidet die ausgewählte Kurve am Startpunkt oder anders herum, der Startpunkt definiert sowohl die Lage der Achse, wie auch die ausgewählte Kurve. Als 'Achse' ist auch ein gerader Abschnitt zu verstehen, der ausreichend lang ist (Anspruch 2).

Um den Startpunkt herum wird ein Steuerbereich (als Navigationsabschnitt oder -fenster) deutlich gemacht, den der Benutzer "als optisch hervorgehoben" wahrnimmt. Diese Wirkung ist auf mehreren Wegen erreichbar, andere Farbe, anderer Kontrast, stärkere Linie oder andere Linienform (Punkte, gestrichelt, usw.).

Für die optische Hervorhebung gibt es mehrere Varianten.

Es können eine Obergrenze und eine Untergrenze hervorgehoben werden (durch Symbole, wie Kreise, Punkte, Rechtecke oder Sterne), es kann der Steuerbereich durch Verdickung auf der geraden Achse hervorgehoben werden oder dieser Steuerbereich wird andersfarbig dargestellt, als der übrige Abschnitt der geraden Achse. Oder aber die Abschnitte außerhalb werden heller oder "ausgegraut" (im Grauwert kontrastärmer oder schwächer) dargestellt.

Mit der "wirkungsmäßigen optischen Hervorhebung" dieses Steuerbereichs (Abschnitts) wird dem Benutzer die Vielfalt von in der Datenbank gespeicherten Lösungen visualisiert. Er erhält eine Möglichkeit, diese Vielfalt in ihrer Darstellung **zu steuern** und für die Darstellung auszuwählen. Deshalb wird von einer "steuerbaren Visualisierung" gesprochen, die nicht nur eine Darstellung als solches ist, sondern dem Benutzer die Möglichkeit gibt, die Darstellung so zu modifizieren (zu steuern), dass er in der Lösungsvielfalt, die in der Datenbank gespeichert ist, über das angezeigte DVH-Diagramm navigieren kann. Eine mächtige Steuerung über einen unscheinbaren DVH-Diagrammabschnitt, der indes gefühlsmäßig vom Benutzer/Planer verstanden wird.

Die Vielzahl von derzeit nicht dargestellten Lösungen (alle Lösungen, die gespeichert sind, abzüglich der einen, im Hauptdiagramm dargestellten Lösung) kann so visualisiert werden. Sie wird noch nicht dargestellt, da nur eine der Vielzahl von Lösungen im Hauptdiagramm darstellt ist, aber sie wird angedeutet durch den Steuerbereich, der um den genannten Startpunkt herum liegt und - optisch wirkend - hervorgehoben wurde.

Dazu ist auch zu sagen, dass die ausgewählte DVH-Kurve (im Folgenden auch Graph oder Funktion genannt) diejenige ist, welche die DVH-Kurven in den gespeicherten, derzeit nicht dargestellten Lösungen, festlegt. Diese sind die der ausgewählten DVH-Kurve entsprechenden DVH-Kurven. All diese Kurven schneiden die festgelegte gerade Achse ebenfalls, und alle diese Schnittpunkte definieren den hervorgehobenen Bereich.

Eine Möglichkeit, diesen Bereich oben und unten zu begrenzen, ist es, den größten und den kleinsten Wert des Endwerts zu nehmen und diesen Abschnitt hervorzuheben (obere Grenze, untere Grenze, Hervorhebung, andersfarbige Darstellung). Damit ist in dem "erster Steuerbereich" genannten, hervorgehobenen Bereich (auch: Navigationsabschnitt) die Lösungsvielfalt repräsentiert, bezogen auf die ausgewählte DVH-Kurve.

Es versteht sich, dass auch andere DVH-Kurven aus der Kurvenschar ausgewählt werden können, bei denen die Hervorhebung des ersten interaktiven Steuerbereichs in gleicher Weise erfolgt. Eine Mehrzahl von Auswahlen kann erfolgen, so dass mehrere DVH-Kurven mehrere hervorgehobene Bereiche aufweisen, die jeweils auf einer anderen geraden Achse liegen (Anspruch 6).

Bevorzugt liegen alle diese Achsen(abschnitte) parallel und bevorzugt sind auch alle diese Achsen(abschnitte) vertikal orientiert. In weiterer Ausgestaltung können einzelne Achsen(abschnitte) auch in horizontaler Richtung orientiert sein.

Jeder hervorgehobene Bereich bildet die Vielzahl von Lösungen in der Datenbank ab, die dort gespeichert für den Benutzer aufwarten, die aber noch nicht als die jeweilige Lösung vollständig in dem DVH-Diagramm abgebildet sind. Der Benutzer hat es damit an der Hand, ausgehend von einer (oder mehreren) DVH-Kurven die Lösungsvielfalt ins Blickfeld zu nehmen, mit besonderer Betrachtung einer der Kurven des DVH-Diagramms, wobei diese eine Kurve aus der Kurvenschar einem der Volumina entspricht. In einem Beispiel kann das Target T betrachtet werden und die Lösungsvielfalt anhand der Target-DVH-Kurve bewertet werden.

Die Bewertung selbst ist nicht Gegenstand des Anspruchs 1, sondern der Anspruch umfasst die interaktive Auswahl mit der Darstellung, die eine solche (menschliche) Bewertung ermöglicht. Um diese Bewertung überhaupt vornehmen zu können, bedarf es einer verständlichen, analytischen und übersichtlichen Darstellung, die den Betrachter nicht überfordert, ihm aber ausreichend Informationen an die Hand gibt, um Entscheidungen zu treffen. Eine klare technische Herausforderung.

Ausgestaltungen der jeweiligen Erfindung finden sich in den abhängigen Ansprüchen.

Dazu gehören die Nachberechnung von Zwischenlösungen (sog. Rekombination, Ansprüche 10, 15, 16) oder eine Extension (Anspruch 11), welche den Steuerbereich nach oben oder unten (bei vertikalen geraden Achsen) erweitert.

Die Bedienelemente helfen bei der Interaktion mit dem Benutzer. Ein Selektor bildet einen Schnittpunkt auf der geraden Achse und kann mit einem Mauszeiger gehandhabt werden. Ein Restriktor verkleinert den Steuerbereich (er wird in Längsrichtung verkürzt).

Die Ausgestaltung (vgl. Anspruch 11) benennt auch die übrigen DVH-Kurven, die auch dargestellt werden.

Der Restriktor kann nicht über den aktuellen Schnittpunkt hinausbewegt werden und damit liegt der Start- oder Schnittpunkt immer zwingend im Steuerbereich, Anspruch 14.

Die Verlagerung des beanspruchten weiteren Bedienelementes führt zur Einschränkung des ersten Steuerbereichs und damit Ausschluss von Lösungen zur Aktualisierung der ersten Steuerbereiche (wenn mehrere vorgesehen sind) auf allen geraden Achsen mit einer Entfernung der Schnittpunkte der ausgeschlossenen Lösungen, Anspruch 18.

Die Bedienelemente erlauben die Steuerung der Veränderung der Darstellung. Die dargestellte Lösung wird zu einer anderen dargestellten Lösung geändert, wenn beispielsweise der Selektor als Startpunkt oder als Schnittpunkt einer DVH-Kurve mit einer geraden Achse verändert wird. Die anderen DVH-Kurven folgen dieser Veränderung indem sie zu der Kurven als Lösung zugehörig sind, und dargestellt werden, der aktuell mit dem Selektor eingestellt ist (zur Darstellung).

Ausführungs**beispiele** der beanspruchten Erfindung werden anhand der Figuren erläutert.

| | |
|---|---|
| **Figur 1** | ist ein Systemaufbau mit Komponenten des Computersystems, welches die Darstellungseinrichtung 20 steuert. Auf der Darstellungseinrichtung 20 ist ein DVH-Diagramm 30 im unteren Abschnitt des Bildschirms repräsentiert, dessen Kurvenschar 39 die Vielzahl von Kurven (auch Graphen genannt) repräsentiert, die im DVH-Diagramm 30 aufgetragen sind und die einer einzigen Lösung zugehörig sind, von denen eine Vielzahl von Lösungen in der Datenbank 10 gespeichert ist. |
| **Figur 2** | ist ein Ausschnitt aus der Bildschirmdarstellung 20 von Figur 1 mit dem DVH-Diagramm 30. Hier ist die Kurvenschar 39 zu sehen und auch die Hilfsdarstellungen 35,36 und 37 als drei senkrecht aufeinander stehende Schnitte. |
| **Figur 3** | ist das DVH-Diagramm 30 der Figur 2 mit den Achsenvisualisierungen von insgesamt vier Planungskriterien, jeweils einem auf den beiden Ohrspeicheldrüsen und den beiden Tumorvolumina, die als Beispiele von betrachteten Volumina anzusehen sind. |
| **Figur 4** | ist eine vereinfachte Darstellung zur Erläuterung des Systems der Figuren 1 bis 3, wobei nur drei Kurven eines DVH-Diagramms 30 gezeigt sind und auch die Ausbildung der betrachteten Volumina R, T und N. Das Volumen R im Zentrum ist das Risiko (ein zu schützendes Organ), der Tumor ist T, im vereinfachten Beispiel hufeisenförmig um das Risiko herumgelegt, und der übrige Bereich außerhalb der beiden beschriebenen Bereiche R und T ist der nicht klas- |

sifizierte Bereich N, der ebenfalls ein Volumen bildet, das in den Kurven der Kurvenschar 39 dargestellt ist. Entsprechend der Vereinfachung in der Form der Volumen sind auch nur drei Kurven im DVH-Diagramm 30 zu sehen, die zugeordnet r (für das Risiko R), n für das nicht klassifizierte Gebiet N und t für das Zielvolumen T (Target) gelten.

**Figur 5**     veranschaulicht die Darstellung von Figur 4, wobei im Volumenbereich der Hilfsdarstellung 35 auch Isodosis-Linien eingezeichnet sind, die zu der technischen Einstellung gehören, welche in dem DVH-Diagramm 30 durch drei Kurvenverläufe repräsentiert ist. Der Abstand der Isodosis-Linien beträgt jeweils 10 Gy. Aufgrund der Zweidimensionalität des betrachteten Falles sind die beiden anderen, senkrecht zur Abbildung 35 orientierten Hilfsbilder 36a, 37a einfache Linien. Ihnen ist keine Aussagekraft zuzuordnen, was eine Folge der Vereinfachung zur Erläuterung des Systems ist.

**Figur 6**     ist die Darstellung von Figur 5, bei der mit der Steuerung der Darstellung begonnen wird, orientiert an einer Festlegung eines Punkts 41a auf der Kurve n. Die Notation r, n und t wird hier verlassen, zugunsten der ausgewählten Kurve 40a und der zu dieser Lösung gehörenden anderen Kurven 50a und 60a, die jeweils Bestandteil einer jeweiligen Kurvenschar 40,50,60 sind, welche die gespeicherte Vielfalt von Lösungen in der Datenbank 10 bereithält.

**Figur 7**     ist eine Darstellung des DVH-Diagramms aus Figur 6, bei dem die vertikal ausgerichtete, gerade Achse 71 vorbereitet ist, gedreht zu werden. Dies um einen Drehpunkt 71a*.

**Figur 8**     ist der Zustand der Figur 7 nach der Drehung der geraden Achse 71 zum Erhalt einer horizontal verlaufenden, geraden Achse 73, die parallel zur Dosis-Achse in x-Richtung verläuft.

**Figur 9**     ist die Darstellung aus Figur 8 mit einem entsprechenden Steuerbereich 74, der um den Schnittpunkt 41a gelegen ist und auf der horizontal verlaufenden, geraden Achse 73 zwischen 73a und 73b gebildet wird.

**Figur 10**     ist die Abbildung aus Figur 6 in einem anderen Modus "Hinzufügen einer Achse" (=add), der aktiviert ist.

**Figur 11**     ist die hinzugefügte neue, gerade Achse, die in vertikaler Richtung ausgerichtet ist und den DVH-Graphen 60a bei 65 Gy schneidet. Der Schnittpunkt 61a eröffnet einen neuen Steuerbereich 76.

**Figur 12**     veranschaulicht den Modus "Navigation" (=Nav) und reduziert den Steuerbereich 76 von Figur 11 auf einen kleineren Steuerbereich 76a, mit Blick auf den DVH-Graphen 60a.

**Figur 13**     veranschaulicht eine Reduzierung des Steuerbereichs 72 aus Figur 12 durch ein auf der geraden Achse 71 nach unten verschobenes, oberes Bedienelement 71e, das "Restriktor" genannt werden soll, weil es den oberen Endwert des Steuerbereichs 72 reduziert und einen kleineren Steuerbereich 72a festlegt.

**Figur 14**     zeigt eine schon hinzugefügte dritte gerade Achse, die vertikal ausgerichtet ist. Diese gerade Achse 77 ist dem dritten Graphen 50a im DVH-Diagramm 30 zugeordnet.

**Figur 15**     veranschaulicht die Darstellung eines neuen Plans (Plan 'b'), der durch Verlagerung des Punktes 61a zum Punkt 61b im Steuerbereich 76a ausgewählt wird und dann dargestellt worden ist.

**Figur 15a**     veranschaulicht ein Systembild eines Abschnitts einer vertikal ausgerichteten geraden Achse 70 zwischen den Werten 97,5% und 98%, wobei zusätzlich zu den in der Datenbank gespei-cherten vorberechneten Lösungen $a$, $a_1$, $a_2$ weitere Lösungen $d_1$, $d_2$... $d_{10}$ durch Nachbe-rechnen im Sinne einer "Rekombination" als zusätzliche Stützwerte auf der geraden Achse 70 hinzugefügt werden.

**Figuren 15b, 15c, 15d**     veranschaulichen in einer Ablauffolge von mehreren Schaubildern, wie aus wenigen gespeichert und vorberechneten Lösungen a, b, c eine Mehrzahl von Lösungen wird, die als Zwischenstellen (Stützwerte) wirken. Dies zwischen den vorberechneten Lösungen, um die bestehenden Lücken aufzufüllen und damit eine gefühlte Kontinuität der Navigation zu erreichen.

| | |
|---|---|
| **Figur 16** | zeigt eine Folgefigur aus Figur 15, bei der der untere Grenzwert (untere Restriktor 75f) auf der dem Tumor zugeordneten geraden Achse 75 aufwärts verändert wird. Das Steuerintervall 76a wird nochmals kleiner und hat nur mehr eine Länge 76b. |
| **Figur 17** | ist die Darstellung eines Ausgangspunkts zur Entfernung einer Achse (Modus 'remove'). Der Modus ist mit dem Button 23 auf eine solche Achsenentfernung eingestellt. Zu entfernen ist die Achse 75. |
| **Figur 18** | veranschaulicht die Figur 17 mit entfernter gerader Achse 75, wobei auch die von ihr vorgegebenen Einschränkungen durch den engen Steuerbereich 76b wegfallen. |
| **Figur 19** | zeigt den Beginn eines weiteren möglichen Navigationsschritts, der "Extension". Der Steuerbereich 72b soll erweitert werden, auf einen Bereich unterhalb des unteren Endes 71f dieses Steuerbereichs 72b. |
| **Figur 20** | veranschaulicht die Verlängerung des Steuerbereichs 72b zu einer noch nicht durchgehenden größeren Länge 72c (noch offen zwischen 41b und 71g). |
| **Figur 21** | veranschaulicht den hinzugekommenen Abschnitt 72b', der jetzt auch zu einer Veränderung des Schnittpunktes 41b herangezogen werden kann, indem dieser Schnittpunkt 41b als "Selektor" in Abwärtsrichtung verändert werden kann, wodurch andere Lösungen dargestellt werden, die dem Benutzer aber suggerieren, dass sie durch Verschieben zustande kommen. |
| **Figur 22** | zeigt eine neu berechnete, dritte Lösung mit den angezeigten/dargestellten Graphen 40c, 50c und 60c, die durch eine Abwärts-Veränderung des Punktes 41b zum Punkt 41c entsteht und abgebildet wird, statt der zuvor dargestellten Lösung aus Figur 21 mit den Graphen 40b, 50b und 60b. Bei den neuen Graphen 40c, 50c, 60c handelt es sich um eine durch Rekombination entstandene neue Lösung mit dem Schnittpunkt 41c auf der geraden Achse. |

**Figur 1** veranschaulicht das System, welches hinter der Bildschirmdarstellung steht. Das System S (links in Figur 1) ist die Steuerungstechnik, die für die Darstellungseinrichtung 20, so ein Bildschirm, ein Tablet oder ein anderweitiger tragbarer PC, oder eine über einen Beamer an eine Leinwand geworfene Bildschirmdarstellung, verantwortlich ist.

Die Darstellungseinrichtung 20 ist insoweit durch Ausführungsbeispiele ausgefüllt, als ein Benutzer visuell erfahren soll, was die Datenbank 10 an vor-berechneten Lösung beinhaltet, von denen jede einen eigenen Plan mit einer Vielzahl von technischen Parametern enthält, der einer Therapieeinrichtung mit einem Strahlenkopf vorgegeben werden kann, wo diese technischen Parameter dann auch Auswirkungen haben.

Wie die Therapie selbst erfolgt, ist nicht Gegenstand dieser Beschreibung, sondern Gegenstand ist die Art und Weise der Handhabung der technischen Parameter, die repräsentativ für einen jeweiligen Therapieplan stehen, als solches aber reine technische Einstellgrößen eines technischen Geräts sind, das mit technischen Parametern arbeitet und entsprechende Motoren, Einstellknöpfe und Stellglieder besitzt, die die technischen Parameter, wenn sie einmal eingestellt sind, exakt nachführen.

Die Darstellungseinrichtung 20 als hier dargestellter Bildschirm hat einen Darstellungsbereich, auf dem vier Abschnitte zu sehen sind. Ein unterer, größerer und rechteckig gestalteter erster Bereich 30 ist der DVH-Bereich, der eine Dosis-Volumen-Histogramm-Darstellung visualisiert. Dargestellt sind zwei Achsen, X- und Y als Dosisachse (in X-Richtung) und als Volumenprozent-Achse (in Y-Richtung). Symbolisch dargestellt sind eine Vielzahl von Graphen 39, die Dosis-Volumen-Graphen einer Darstellung sind, und in ihrer Anzahl derjenigen Anzahl entsprechen, die als definierte Volumina in einem Zielgebiet festgelegt worden sind. Oberhalb der größeren Dosis-Volumen-Darstellung 30 sind drei Hilfsdarstellungen angeordnet, die ein Beispiel darstellen. Diese drei Hilfsdarstellungen 35, 36 und 37 sind im Beispiel drei Schnitte, wie sie an Figur 2 veranschaulicht werden, in horizontaler, vertikaler und dazu senkrechter vertikaler Richtung, bezogen auf das Zielgebiet, in dem die definierten Volumina liegen. Zumindest eines dieser Volumina ist ein Target, das durch einen Tumor repräsentiert wird. Zumindest ein Volumen ist ein Risiko, welches beispielsweise von einem empfindlichen Organ oder einem empfindlichen Gewebebereich gebildet wird. Ein weiteres Volumen kann (muss aber nicht) als ein Umfeld-Volumen oder ein nicht klassifiziertes Volumen definiert werden.

Die Steuerung der graphischen Darstellung arbeitet mit der Steuereinrichtung S, die aus einem Visualisierungs-Modul 11 besteht, welches nach Art eines Interpreters und meist auch mit einem Cache arbeitet, der die aus der Datenbank 10 über den Datenpfad 15 ausgelesenen Lösungen zwischenspeichert. Die Lösungen werden dabei so aufbereitet, dass sie als Bildschirmsignal über die Verbindungsleitung 11a der Darstellungseinrichtung 20 zugeführt. Das Bildschirmsignal ergibt die Darstellung, die schematisch auf der Bildschirmeinrichtung 20 aufgetragen ist.

Das Visualisierungs-Modul 11 gibt die Lösungen entweder aus der Datenbank 10 oder aus seinem eigenen internen Cache an die Bildschirmeinrichtung 20. Ist eine angeforderte Lösung nicht vorhanden, fordert das Visualisierungs-Modul 11 über die Leitung 11b einen neuen Plan an. Der neue Plan wird von dem Berechnungsmodul 12 erstellt. Diese Berechnung erfolgt durch Verwendung von schon vorberechneten, gespeicherten Plänen in der Datenbank 10, die über die Zugriffsleitung 14 ausgelesen wird. Das Berechnungsmodul ermittelt die neuen Pläne, die "rekombinierte" Lösungen sein können oder ganz neue Lösungen, die nicht "zwischen" schon vorberechneten, in der Datenbank gespeicherten Lösungen liegen, sondern außerhalb.

Diese neuen Lösungen können entweder in einem eigenen Zwischenspeicher der Berechnungseinheit 12 zwischengespeichert werden, sie können über die Speicherleitung 13 in die Datenbank hineingespeichert werden, von wo das Visualisierungs-Modul 11 sie über die Zugriffsleitung 15 auslesen und der Darstellungseinrichtung 20 zuführen kann.

In einer gesonderten, nicht dargestellten Ausführung kann das Berechnungsmodul 12 auch Zwischenlösungen errechnen, die zwischen zwei oder zwischen noch mehreren existierenden Lösungen liegen, die nicht gesondert in der Datenbank 10 zu speichern sind, sondern auf direktem Weg 13a dem Visualisierungs-Modul 11 zugespeist werden, um für die Darstellung an Geschwindigkeit zu gewinnen.

Die Bildschirmleitung 11a ist bidirektional ausgelegt, um Anforderungen, die vom Bildschirm eingegeben werden, in dem Visualisierungs-Modul 11 auszuwerten und entsprechende Pläne aus der Datenbank abzurufen oder von dem Berechnungsmodul 12 errechnen zu lassen.

Es werden in der Datenbank eine Vielzahl von Plänen, zumindest 20, meist aber mehrere hundert Pläne vorgehalten, die als fertige Lösungen in einem hier nicht dargestellten Berechnungssystem vorberechnet worden sind und mehrkriteriell (MCO) optimierte Lösungen darstellen können. Für die Erlangung solcher Pläne soll auf die eingangs verwiesene US-Patentschrift verwiesen werden, die auch anderweitige Fundstellen nennt, wie diese vorberechneten Pläne erstellt, erhalten und gespeichert werden.

Die Steuerung der Darstellungseinrichtung 20 und die Steuerung der Darstellung der Lösungsvielfalt in der Datenbank soll Gegenstand der weiteren Figuren sein. Diese Steuerung der Vielfalt der Lösungsmöglichkeiten kann auch so verstanden werden, dass eine Navigation ermöglicht werden soll, die interaktiv arbeitet. Das beschriebene System ist ebenso ein Planungswerkzeug zur interaktiven Auswahl von passenden Lösungen im Sinne von Therapieplänen, die sich jeweils aus einer Anzahl von technischen Steuerparametern zusammensetzen. Für die Navigation werden die Graphen verwendet, die auf dem Bildschirm 20, insbesondere im Bereich des Dosis-Volumen-Histogramms 30 aufgetragen sind und die mit 39 in ihrer Gesamtheit repräsentiert werden.

Eine genauere Darstellung dieser mehreren Dosis-Volumen-Graphen 39 zeigt **die Figur 2.**

Hier ist der Abschnitt 30 an der unteren Bildschirmhälfte zu erkennen. Vertikal ist die Achse für die Volumenprozente und horizontal die Dosisachse aufgetragen. Es sind eine Vielzahl von Graphen zu ersehen, von denen einige sehr stark und sehr früh abfallen und andere sehr spät erst abfallen. Die rechten, spät abfallenden Graphen sind diejenigen, die für die unerwünschten Gewebezonen, also das Target (Tumor) vorgesehen sind. Die früh abfallenden Graphen sind für die gefährdeten Risikoorgane. Die Organe sind in den drei Schnittdarstellungen 35, 36, 37 in drei zueinander senkrechten Schnitten dargestellt. Sie sind dort durch Konturlinien (auch: Umrisslinien) gekennzeichnet. Ebenfalls dargestellt sind Isodosen (Linien gleicher Dosis), die sich in den drei Schnittansichten ergeben. Durch Auswahlknöpfe und Auswahlzonen können verschiedene Schichten (Slices) ausgewählt werden, die in den drei Ebenen verfügbar sind. Im linken Bild 35 als erstes Hilfsbild ist die transversale Ansicht zu sehen. Dort ist Slice 58 dargestellt mit einem Vergrößerungsfaktor von 1,5. Im mittleren, oberen Hilfsbild 36 ist eine sagittale Ansicht zu sehen, mit dem Slice 234 und einer Vergrößerung von 1,5. Im rechten Hilfsbild 37 ist die Frontalansicht zu sehen, es ist Slice 289 bei einer Vergrößerung von 1,5.

Am unteren Bildrand sind Modusauswahl-Selektoren gezeigt, die den Modus umschalten, in dem sich die Navigation im Bereich 30 befindet. Es kann eine Navigation freigeschaltet werden mit dem Auswahlselektor 22, es kann die Hinzufügung einer Achse definiert werden mit dem Auswahlselektor 21, und es kann das Löschen einer Achse vorgegeben werden mit dem Auswahlselektor 23.

Die Arbeitsweise dieser Moduswahl wird im Folgenden beschrieben.

Eine erste Übersicht gibt die Figur 3, die im Folgenden vertieft wird und auch vereinfacht wird, um die Erläuterung zu verdeutlichen. Sinngemäß können aber die Vielzahl von Dosis-Volumen-Graphen 39 auch auf die anderen Bilder übertragen werden, wo für die weitere Erläuterung nur von drei Graphen ausgegangen wird, bei einer vereinfachten Gestalt der zu betrachtenden Volumina im linken oberen Hilfsbild.

**Die Figur 3** zeigt die gleichen Ansichten wie die Figur 2, nur sind zusätzlich vertikale gerade Achsen 70 eingezeichnet, von denen jede zumindest einen der Dosis-Volumen-Graphen schneidet, unter Bildung eines jeweiligen Schnittpunktes. Unterhalb und oberhalb eines Schnittpunktes, also "um einen jeweiligen Schnittpunkt herum", ist ein Bereich durch oberen und unteren Grenzwert definiert oder gezeigt, der sich als Steuerbereich darstellt, mit dem eine Navigation erfolgt, wie das im Folgenden näher erläutert wird.

Es wäre anzumerken, dass die drei dargestellten oberen Hilfsbilder 35, 36 und 37 auch andere Darstellungsweisen sein können, beispielsweise eine dreidimensionale Ansicht in nur einem Bild, welches rotierbar, drehbar oder kippbar in verschiedenen Achsen ist, um die im Dosis-Volumen-Histogramm 30 eingestellte Lösung anderweitig dem Benutzer zu vermitteln oder visuell darzustellen. Die Navigation, die im Folgenden erläutert wird, ist zu sehen an den Steuerbereichen, die als ein Längenabschnitt auf der vertikalen Achse 70 eingezeichnet sind, um einen jeweiligen Schnittpunkt mit einem Dosisgraphen herum.

Die Steuerabschnitte 70a sind wirkungsmäßig optisch hervorgehoben, relativ zum Rest der geraden Achse 70, und sie visualisieren die Lösungsvielfalt, die nicht dargestellt wird, aber präsent ist. Sie ist präsent in der Datenbank 10 und kann über das Visualisierungs-Modul 11 unmittelbar auf dem Bildschirm 20 in dem Dosis-Volumen-Histogramm 30 dargestellt werden, wenn der Schnittpunkt, der auch Selektor heißt, "verschoben" wird, innerhalb des Steuerbereichs 70a, der die obere und untere Grenze für eine Verschiebung definiert. Die obere und untere Grenze können ebenfalls verschoben werden (auch benannt: Restriktor), eine weitere Art der Navigation. Dadurch wird der Steuerbereich 70a kleiner. Eine weitere Art der Navigation ist die Veränderung des kleineren Steuerbereichs 70a in einen neuen Steuerabschnitt hinein, also oberhalb des oberen Endwertes und/oder unterhalb des unteren Endwertes, die bislang nicht für eine Veränderung der Position des Selektors zur Verfügung standen, aber ein Schnittpunkt zwischen dem neu berechneten Dosis-Graphen und der vertikalen, geraden Achse 70 sein wird. Diese Erweiterung wird Extension genannt.

Die visualisierte Lösungsvielfalt kann auch durch "rekombinierte" Lösungen ergänzt werden, die auch präsent sind aber als zwischen in der Datenbank 10 gespeicherten Lösungen liegend neu berechnet werden. Sie können die Steuerabschnitte 70a mit weiteren Werten auffüllen, wie es später erläutert werden wird.

Die skizzierte Navigation in einem oder mehreren Steuerbereichen 70a, die deshalb auch Navigationsabschnitte oder Navigationsfenster genannt werden, ergibt sich aus der vereinfachten Darstellung der folgenden Figuren.

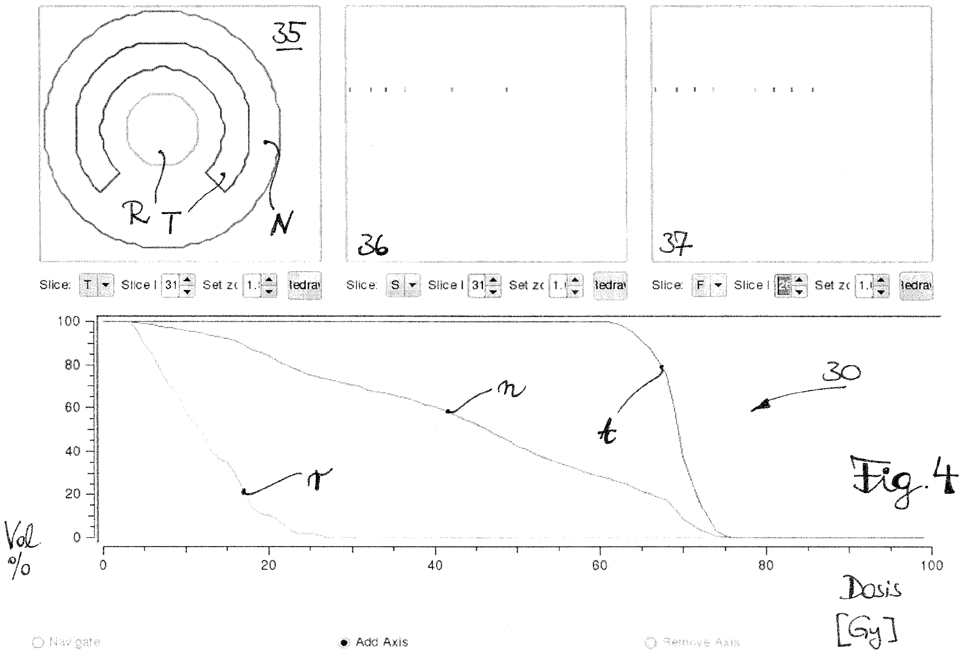

Die Vereinfachung ist anhand **der Figur 4** erläutert. Dazu wird das Volumen nur als flächig angenommen, also zweidimensional, wie im linken oberen Teilbild (oder auch Hilfsbild genannt) gezeigt. In Abschnitt 35 sind drei Volumina gezeigt, das Risiko R im Zentrum, das hufeisenförmig darum herumliegende Target T und der nicht klassifizierte Bereich N dazwischen. Die anderen beiden Schnittdarstellungen, senkrecht dazu, ergeben jeweils eine Linie (oder einige Punkte auf einer Linie), die keine Aussagekraft besitzt. Zugehörig zu den definierten Volumenbereichen der flachen Scheibe aus Hilfsbild 35 sind die drei Dosis-Graphen in dem DVH-Diagramm 30: Der linke untere, früh abfallende Graph r repräsentiert das Risiko. Der mittlere, im Wesentlichen 45° von links oben nach rechts unten verlaufende Graph n repräsentiert das nicht klassifizierte Gebiet N, welches außerhalb des Risikos und außerhalb des Targets liegt, wie im Hilfsbild 35 gezeigt. Der erst spät abfallende, über 100% weit nach rechts reichende Graph bis zu 60 Gy ist t und repräsentiert die Dosis für das Target T. Diese drei Graphen sind dem betrachteten Volumen R, T und N zugeordnet. Sie repräsentieren eine Lösung, die komplett in Figur 4 im Bereich des DVH-Diagramms dargestellt wird. Andere Lösungen sind nicht dargestellt, sie befinden sich in der Datenbank 10 der Steuervorrichtung S für die Bildschirmdarstellung.

In Figur 4 ist die Darstellung vorbereitet, eine Achse hinzuzufügen (Modus: add), die als eine gerade Achse 71, entsprechend den Achsen 70 aus Figuren 2/3 so eingefügt wird, dass sie eine parallel zur Y-Achse der Volumenprozente verlaufende Gerade bildet.

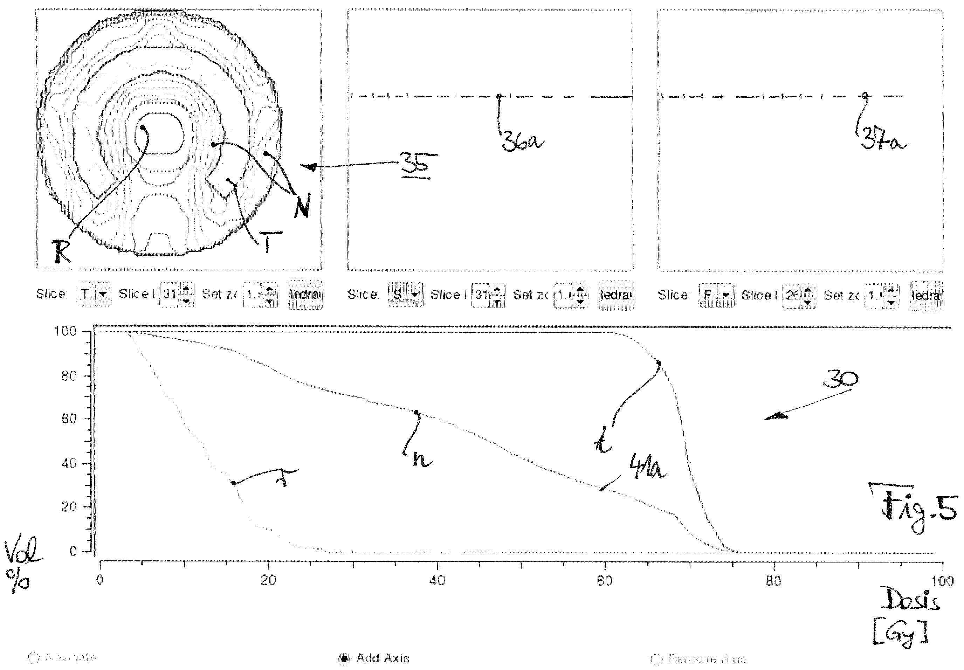

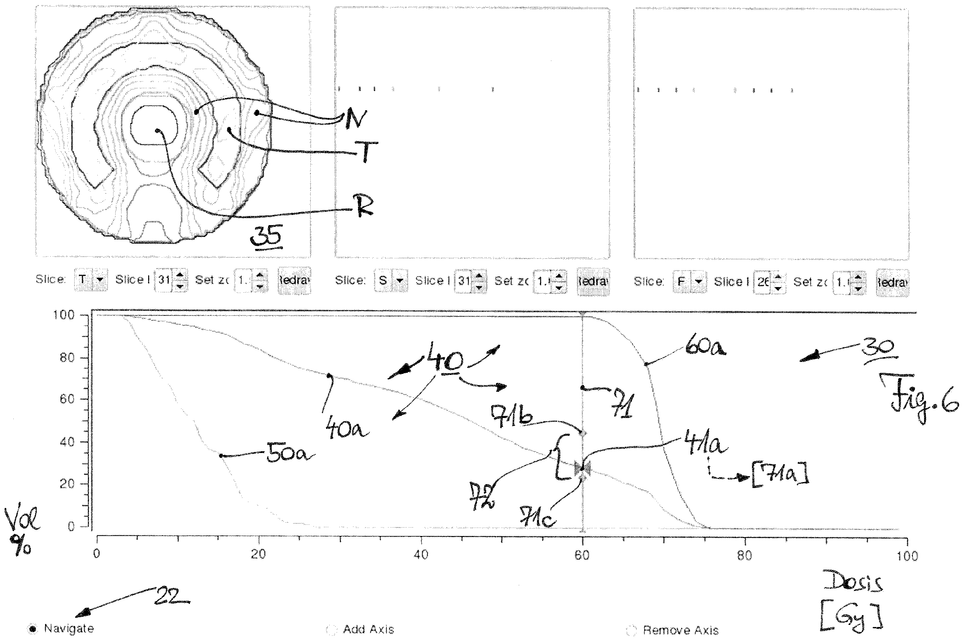

Dies soll an der Figur 6 erläutert werden. Zuvor ist noch mit Figur 5 zu sehen, dass die zwei Linien 36a und 37a senkrechte Schnitte zum Bild 35 sind. Die eingezeichneten Volumen sind hier mit Isodosis-Linien belegt, die den Graphen r, n und t im DVH-Diagramm 30 entsprechen, das auf der Darstellungseinrichtung 20 aufgetragen oder visuell sichtbar gemacht wird. Auch hier ist das System vorbereitet, eine gerade Achse entsprechend der Achse 70 einzutragen, was anhand der Figur 6 dann exemplarisch erfolgt.

Es sei nochmals angemerkt, dass die drei Graphen der DVH-Diagrammdarstellung 30 nur eine vereinfachte Darstellung ist, die durchaus die Form und Gestalt der Figuren 2 und 3 annehmen kann und im praktischen Fall auch annehmen wird, aber für eine Erklärung der Funktionalität der DVH-Navigation die drei Dosis-Volumen-Graphen und die zugehörigen betrachteten Volumina völlig ausreichend sind.

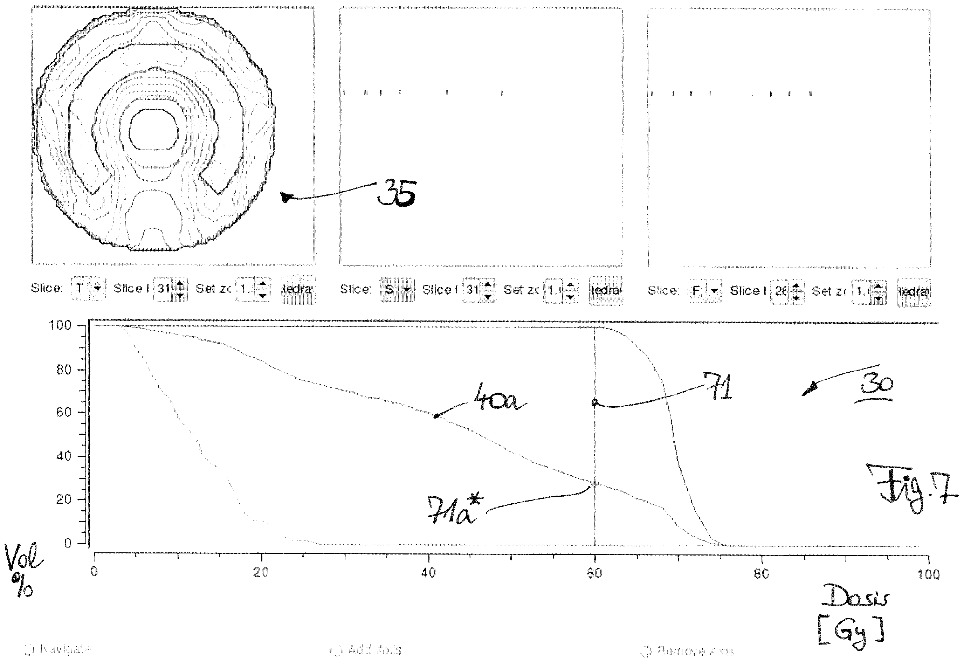

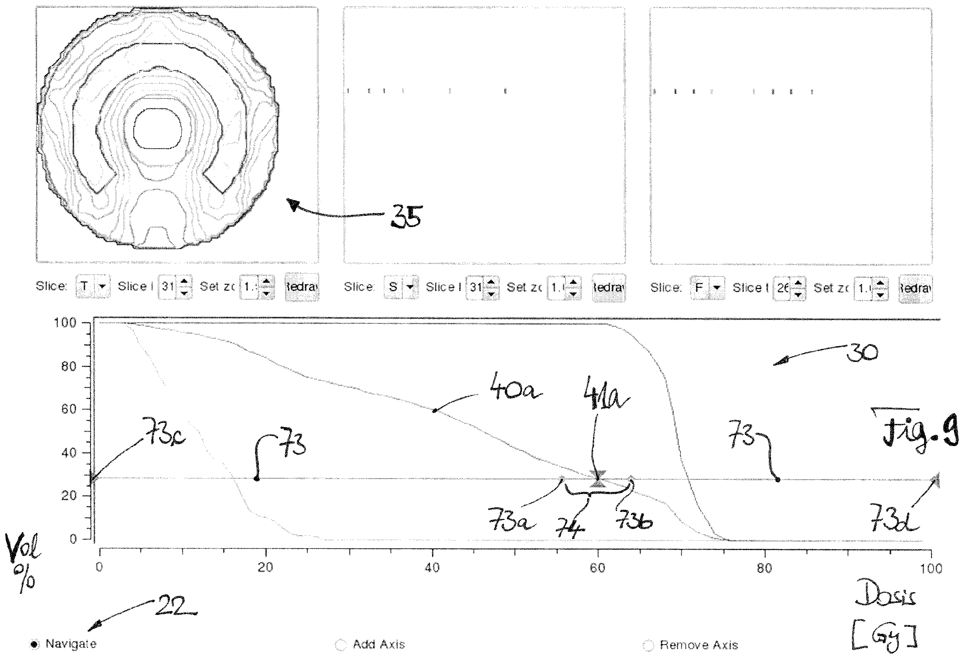

**Die in Figur 6** dargestellte Lösung mit den drei Kurven 40a (nicht klassifiziert), 50a (Risiko) und 60a (Tumor) ist in der Figur 7 ebenfalls dargestellt. **In Figur 7** wird die vertikale Achse 71 modifiziert. Dies kann durch eine Art von Aktivieren erfolgen, die außerhalb der dargestellten Modi 21, 22, 23 (unterer Bildrand) geschieht, beispielsweise durch einen Rechtsklick auf den Schnittpunkt 41a oder auf die Achse 71 selbst. Dadurch wird ein Symbol 71a* dargestellt, welches die Möglichkeit aufzeigt, dass die Achse 71 geschwenkt oder gedreht werden kann. Sie kann beispielsweise so verdreht werden, wie das zur Achse 73 **in Figur 8 und 9** dargestellt ist. Die gerade Achse 71 hat dann als gerade Achse 73 eine horizontale Ausrichtung.

Andere Orientierungen der Achse 71 sind ebenfalls möglich, Zwischenstellungen zwischen 90° und 0°, beispielsweise 40°, 50° oder 60°. Dieser Winkelbereich kann in Plus- oder Minusrichtung eingestellt werden, so dass die vertikale Achse 71 um ±89° (mit jedem Zwischenwert) geschwenkt werden kann. Es ergibt sich dadurch derselbe Schnittpunkt 41a (oder 71a) aus Figur 6, nur mit einer anderen Orientierung der geraden Achse.

Zugehörig ist **in Figur 9** auch ein anderer Steuerbereich 74 eingetragen, der auf der Achse 73 liegt und die in der Datenbank gespeicherte Vielfalt von Lösungen repräsentiert. In entsprechender Übertragung des Bereichs 72 aus Figur 6 ist Bereich 74 in Figur 9 ausgebildet, als Steuerbereich oder "Navigationsabschnitt". Die ausgewählte Lösung, die einzig in dem DVH-Diagramm 30 dargestellt ist, ist diejenige von Figur 6. Der obere größte Wert 71b und der untere kleinste Wert 71c der Kurvenschar 40 (alle Kurven 40a, 40b, 40c und weitere mit Blick auf die gespeicherten, vorberechneten Lösungen), entspricht hier dem linken und dem rechten Wert 73a, 73b. Es ist, mit Blick auf die Dosisachse auch der kleinste bzw. der größte Wert. Mit Blick auf die Fläche des DVH-Diagramms 30 ist es der linke und rechte Wert 73c bzw. 73d. So ist in Figur 6 auch der oberste und unterste Wert der größte und kleinste Wert mit Blick auf die Volumenachse.

Die Abbildung der Figur 9 lädt zur Navigation ein, eingestellt über die Steuerung im Modus-Auswahlselektor 22.

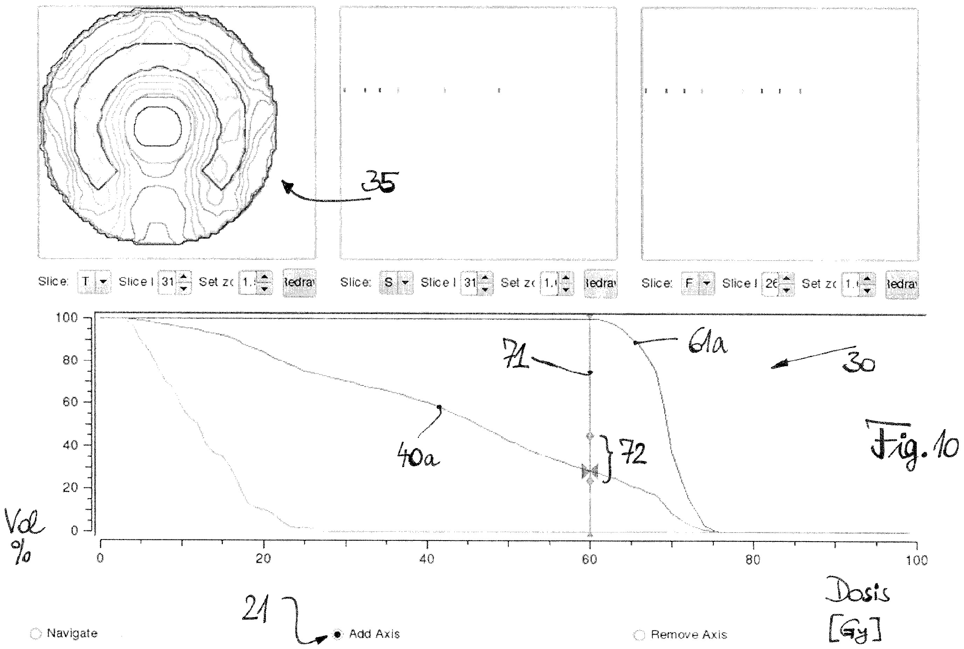

**Mit der Figur 10** kehrt die Abbildung des DVH-Diagramms zurück in die Darstellung von Figur 6. In der Steuerung wird der Modus-Auswahlselektor 21 betätigt, der das Hinzufügen einer neuen Achse erlaubt.

Eine Erläuterung der drei dargestellten Modus-Auswahlselektoren soll hier erfolgen. Der linke Auswahlselektor 22 (als Radio Button) erlaubt das Navigieren innerhalb eines Bereiches, beispielsweise des Steuerbereichs 74 in Figur 9. Das Anklicken oder Aktivieren des Modus-Auswahlselektors 21 erlaubt das Hinzufügen einer weiteren Achse. Diese wird an einem Schnittpunkt definiert, der auf einer der dargestellten drei DVH-Kurven liegt. Die Achsen können auf unterschiedlichen Kurven platziert werden, es können aber auch mehrere Achsen der Gestalt der Achse 71 von Figur 10 auf derselben DVH-Kurve 40a als ausgewählte Kurve platziert werden. Sie sind dann in x-Richtung beabstandet und haben jede einen zugehörigen Steuerbereich, der um den Schnittpunkt herumgelegt hervorgehoben dargestellt ist. Die Hervorhebung repräsentiert die Vielzahl der in der Datenbank 10 gespeicherten Lösungen.

Der dritte Modus-Auswahlselektor ist derjenige, eine Achse zu entfernen. Ist er angewählt, kann eine der in dem DVH-Diagramm platzierten Achsen entfernt werden. Dies wird später erläutert, wenn mehrere gerade Achsen platziert sind.

Im Beispiel der Figur 10 kann die Achse 71 entfernt werden, so dass das Bild der Figur 5 entsteht. Das Entfernen der Achse erfolgt in einem Beispiel so, dass der dritte Modus-Auswahlselektor betätigt wird, und anschließend die Achse dem System verständlich gemacht wird, die zu entfernen ist, beispielsweise durch Anklicken irgendeines Punkts auf der Achse. Die Modus-Auswahlselektoren sind im Beispiel als "Buttons" ausgeführt, können aber auch in anderer Form gestaltet sein, als "Drop down-Menü" oder diese Modus-Auswahlselektoren sind nicht explizit dargestellt und ergeben sich aus einem aufrufbaren Kontext-Menü, das sich beispielsweise öffnet, wenn der Benutzern in dem DVH-Diagramm 30 auf einen neutralen Flächenbereich mit der rechten Maustaste klickt, um das Kontext-Menü zu öffnen und eine der drei beschriebenen Modi anwählt, die Navigation (Nav), das Hinzufügen einer Achse (Add) oder das Entfernen (Remove) einer Achse. Der dann jeweils ausgewählte (aktive) Modus kann in einer nicht dargestellten Ausführungsform an einer gut sichtbaren Stelle des Bildschirms mit Worten als aktiv dargestellt werden. Eine Art der Kennzeichnung ist "jetzt aktiv", folgt: Modus, der eingeschaltet ist (Nav, Add oder Remove).

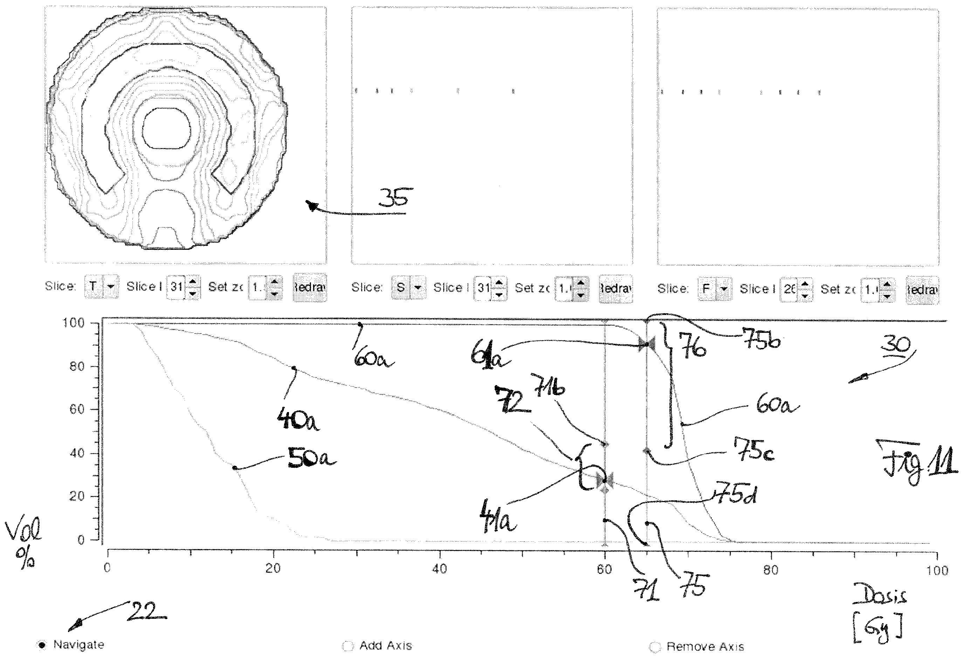

Nach Figur 10 ist der Modus eingeschaltet, eine Achse hinzuzufügen (mit dem Modus-Auswahlselektor 21). Dieser Modus führt zu Figur 11. Diese Figur veranschaulicht die hinzugefügte weitere vertikale Achse 75, die durch Anklicken eines Startpunktes 61a eingeblendet wird. Es entsteht ein Schnittpunkt 61a der DVH-Kurve 60a mit der hinzugefügten Achse 75 bei der in Figur 10 dargestellten Lösung.

Mit dem Festlegen des zweiten Startpunktes 61a, zur Erinnerung, der erste Startpunkt war 41a zur Festlegung der ersten vertikalen Achse 71, wird auch das Navigationsfenster (der Steuerbereich) eröffnet, welches der Bereich 76 ist. Er liegt auf der Achse 75 und hat einen maximalen Wert und einen minimalen Wert, mit Blick auf die Volumenprozent-Achse. Dieser Steuerbereich 76, oder zweiter Navigationsabschnitt (Navigationsfenster) genannt wird, ist größer, als der Steuerbereich 72 von Figur 10. Er liegt im oberen Bereich der Volumenprozent-Achse, weil er die Tumor-Kennlinie betrifft. Der Schnittpunkt 61a sagt, dass mehr als ca. 92% mit einer Strahlendosis von 65 Gy bestrahlt wird, mit Blick auf den Tumor T im linken oberen Hilfsbild.

Der von dem zweiten Steuerbereich 76 aufgespannte Wertebereich der in der Datenbank gespeicherten Lösungen reicht von 44% bis 100% der Volumenprozent-Achse. Der Selektor befindet sich bei 92% und es sind keine Restriktoren eingeschaltet, die später erläutert werden. **In Figur 11** ist der Modus-Auswahlselektor 22 betätigt, so dass eine Navigation als interaktives Auswahlverfahren in den jetzt gebildeten beiden Steuerbereichen 72, 76 möglich ist, die in folgenden Figuren erläutert wird. Beide Steuerbereiche 72, 76 repräsentieren die Vielfalt, respektive die Vielzahl der gespeicherten Lösungen, von denen das Hauptdiagramm 30 nur eine, durch drei Kurven (Graphen) 50a, 40a und 60a repräsentiert ist.

Eine Interaktion erfolgt durch Veränderung der Größe der Steuerbereiche 72 oder 76, oder durch Verlagerung der Schnittpunkte 61a oder 41a innerhalb des jeweiligen Navigationsabschnitts. Beide Handlungen, die Vorgabe der Grenzen der Navigationsabschnitte und die "Verschiebung" des Schnittpunktes innerhalb der (vorgegebenen) Grenzen des Steuerbereichs sind Handlungen zur Navigation und ermöglichen das Auffinden einer Lösung aus der Vielzahl von gespeicherten Lösungen.

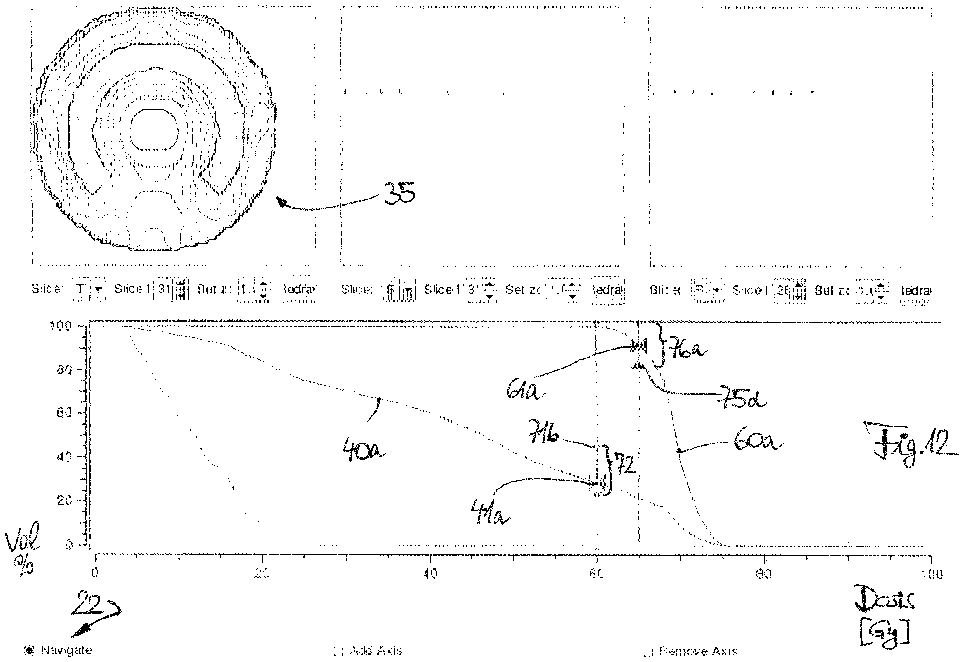

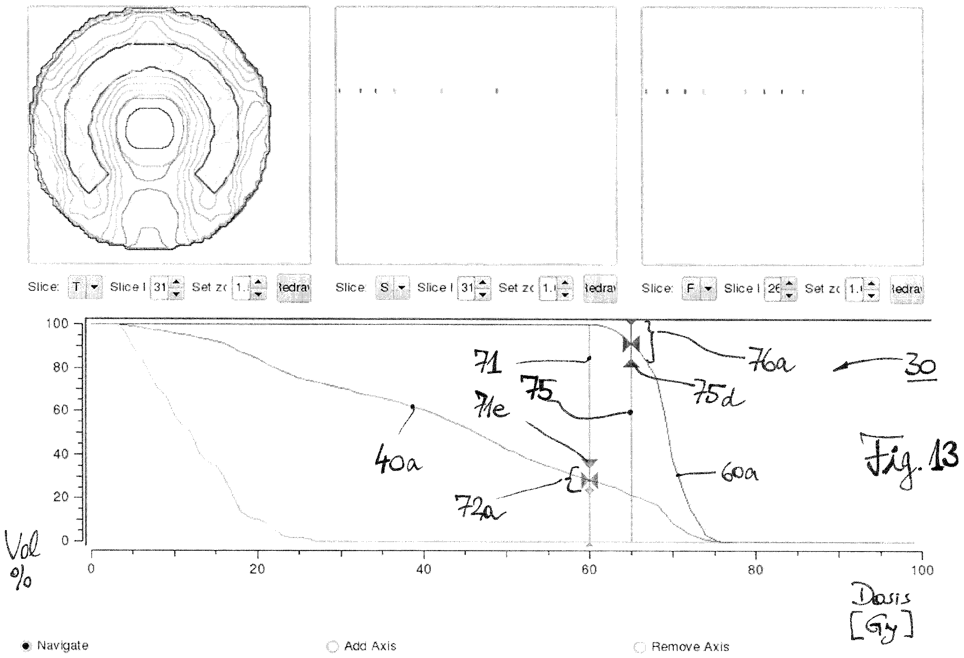

Zwei Interaktionen sind in den **Figuren 12 und 13** gezeigt. Sie bestehen aus dem Verlagern des unteren bzw. des oberen Endwertes des Navigationsabschnitts 76 und 72.

In Figur 12 ist der untere Endwert oder der minimale Wert von dem Wert 75c aus Figur 11 in den Wert 75d in Figur 12 verändert worden. Diese untere Grenze 75d kann entweder aus dem unteren Grenzwert, dem Minimalwert 75c stammen, oder von dem unteren Ende der geraden Achse 75, wie symbolisch in Figur 11 am unteren Rand dieser Achse als vertikal gerichteter Keil ersichtlich. Er wird mit dem Mauszeiger ergriffen und aufwärts auf der Achse geschoben, bis er die Position 75d bei 85% der Volumenprozent-Achse erreicht. Der neu gebildete Navigationsabschnitt hat nur noch die Größe 76a, gegenüber der Größe 76 aus Figur 11. Diese bleibt bei derselben Kurve 60a und auch bei dem gleichen Schnittpunkt 61a, der sich nicht verändert hat.

Entsprechend kann auch der obere Endwert 71b auf der ersten platzierten Achse 71 verschoben werden, und zwar im Beispiel nach unten, was zum neuen Wert 71e von Figur 13 führt. Der dadurch verkleinerte Bereich 72a wird gebildet und hat eine obere Grenze von 34 Volumenprozent.

Im gezeigten Beispiel bleibt durch Veränderung der beiden Grenzen, oben wie unten auf zwei Kurven gemäß den Figuren 12 und 13 die Darstellung auf der jeweils anderen geraden Achse (dem jeweils anderen "Kriterium") unverändert.

Es können aber auch Veränderungen der oberen und unteren Grenze der "NavAbs" (Navigationsabschnitte) so erfolgen, dass eine Veränderung des einen Abschnitts dazu führt, dass zumindest einer oder beide anderen Grenzwert(e) auf den anderen geraden Achsen ebenfalls mitverändert wird/werden.

Diese Veränderung der Grenzwerte auf den anderen Achsen wird dadurch verursacht, dass die auf diesen Achsen gelegenen Schnittpunkte von Lösungen, deren Schnittpunkte außerhalb des veränderten "NavAbs" auf der Ausgangsachse liegen, aus den Navigationsbereichen als NavAbs auf den anderen Achsen entfernt werden. Bildet einer dieser Schnittpunkte einen Grenzwert für einen Navigationsbereich (NavAbs) auf einer anderen Achse, dann führt dessen Entfernung aus dem NavAbs zur genannten Veränderung des Grenzwertes. Dieses Entfernen von Schnittpunkten aus NavAbs auf anderen Achsen erreicht, dass die Veränderung von Startwerten ausschließlich hin zu Lösungen führt, deren Schnittpunkte auf allen Achsen innerhalb der jeweiligen NavAbs liegen.

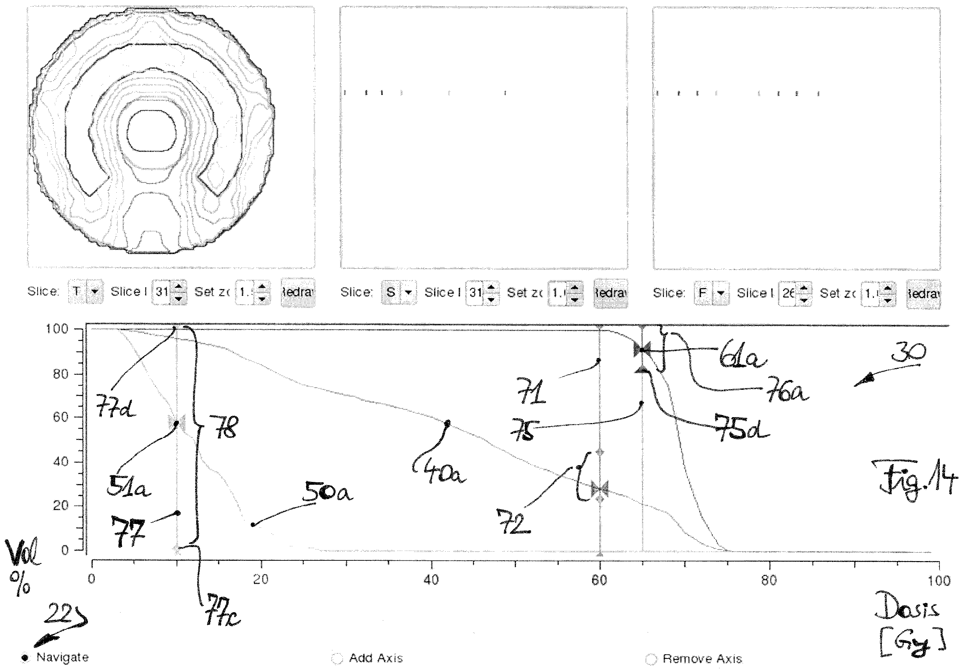

**Figur 14** zeigt die Hinzufügung einer weiteren Achse, wobei zuvor der Modus-Auswahlselektor 21 (Achse hinzufügen) betätigt wurde. Die neu eingeführte Achse 77 wird definiert als vertikale Achse und festgelegt durch Anklicken des Schnittpunktes 51a auf der Kurve 50a. Nachdem diese Kurve die Strahlenbelastung des Risikos R repräsentiert, liegt sie weit im linken Bereich der DVH-Kennlinienschar 39. Der demzufolge aufgespannte Steuerbereich 78 zwischen der oberen und der unteren Grenze, also dem größten und dem kleinsten Wert aller gespeicherten Lösungen für das Risiko R. Der größte Wert ist 77d (bei 100%), der kleinste Wert ist 77c (bei 3%), die vorgegebene Spanne 78 ist ein wenig kleiner als 100%.

Im rechten Abschnitt der Figur 14 ist der Zustand erneut eingestellt, der in Figur 12 rechts zu ersehen ist. Dieser hat einen angehobenen unteren Endwert 75d auf der Target-Kennlinie 60a und keine Begrenzung für den ersten Steuerbereich 72, der durch den Schnittpunkt 41a festgelegt worden ist.

An der Figur 14 ist zu ersehen, dass drei vertikal ausgerichtete gerade Achsen 77, 71 und 75 vorhanden sind, jede zu einer anderen Kurve in DVH-Diagramm 30. Diese Darstellung kann auch modifiziert sein, wenn auf einer Kurve, beispielsweise der zwischen den beiden anderen Kurven liegenden Kurve 40b zwei oder mehr vertikale Achsen platziert werden. Dann sind unterschiedliche Dosiswerte Stellen, die jeweils einen Schnittpunkt verursachen, der zwischen der Kurve 40a und jeder der vertikalen Achsen gebildet wird.

Die in der Beschreibung genannten vertikalen Achsen 71, 77, 75 sind grafisch auf dem Bildschirm 20 aufgetragen, und vom Benutzer über einen Mauszeiger zu handhaben, oder über ein Tablet, bei dem unmittelbar durch Berührung Aktionen an das hinter der Bildschirmdarstellung stehende System S aus Figur 1 gegeben werden. Diese vertikalen Achsen können auch horizontal liegen oder sie können, gemäß vorhergehender Darstellung, geschwenkt werden, um den Schnittpunkt, den sie mit ihrer jeweiligen Kurve des DVH-Diagramms haben.

Die Achsen können auch als Kriterien benannt werden. Ein Kriterium ist in gewisser Weise ein Planungsaspekt, bezüglich dessen ein Benutzer oder Planer, die Qualität des hinter der Darstellung stehenden Plans (von technischen Steuergrößen) zu bewerten vermag. Besonders hilfreich ist dabei die Darstellung im Steuerbereich um den ausgewählten Punkt auf der ausgewählten Kurve. Dieser Steuerbereich (das Navigationsintervall) sagt dem Benutzer, welche Möglichkeiten und welchen Spielraum er hat, den aktuell angezeigten Plan zu ändern, gibt ihm also ein Blick in seine Möglichkeiten und macht die Planungsmöglichkeiten für ihn transparent. Oder dieser Steuerbereich, beispielsweise 72 in Figur 14 oder 76 in Figur 11 zeigt ihm Möglichkeiten auf, die er nutzen kann, wenn er damit befasst ist, den aktuell dargestellten Plan zu ändern.

Der Navigationsabschnitt 76 oder der Abschnitt 72 liegt jeweils auf einer vertikalen Achse oder Linie 71 bzw. 75 und ist damit das oder Bestandteil des Kriteriums, das die Verbesserung eines dargestellten Plans ermöglicht, oder anhand dessen der Benutzer den dargestellten Plan verbessern möchte.

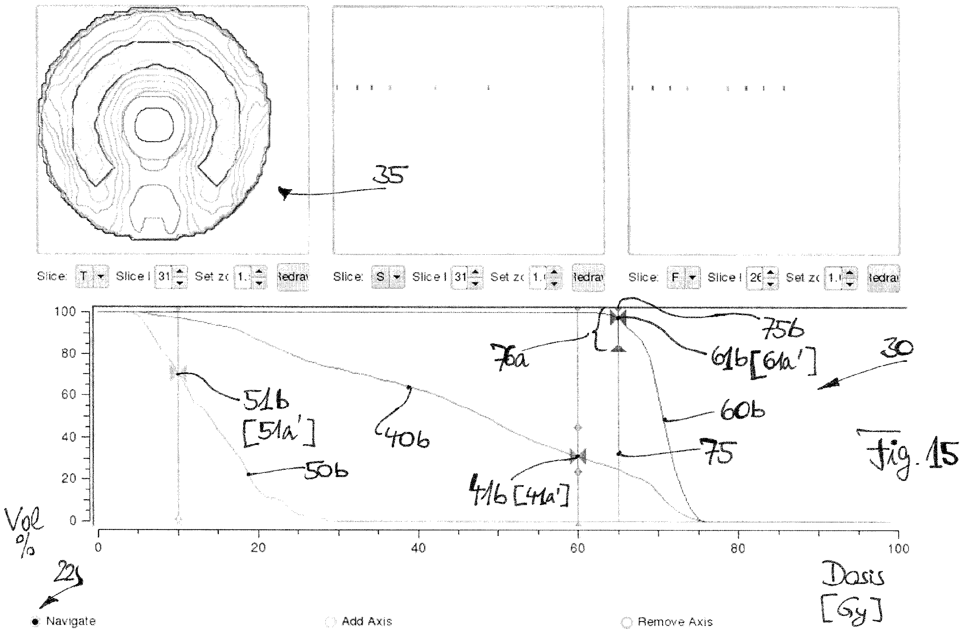

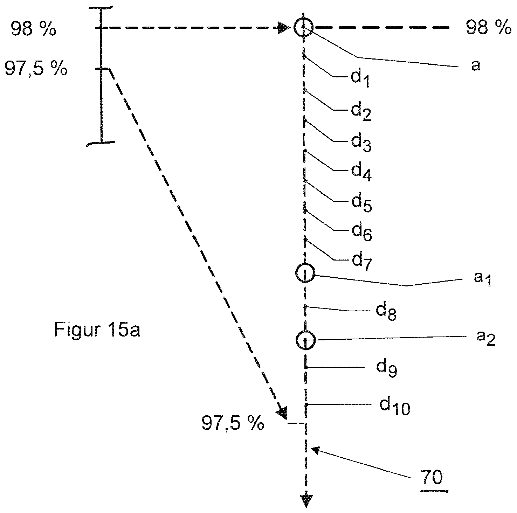

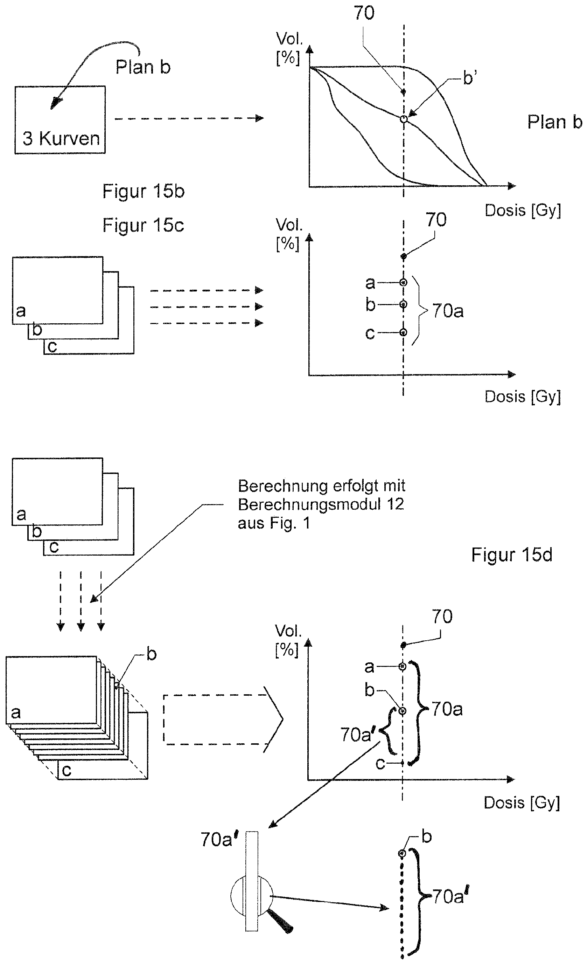

Die Darstellung eines weiteren, neuen Plans, der auch vorberechnet in der Datenbank 10 abgelegt ist, wird anhand der **Figur 15** erläutert. Anhand der Figuren 15a bis 15d wird die Berechnung von neuen Lösungen beschrieben.

Der Startpunkt 61a aus Figur 14 wird nach Aufwärts verschoben. Er wird in Figur 15 zum verschobenen Startpunkt 61b (als 61a' auch dargestellt). Mit ihm wird die Kurve **scheinbar** verschoben, in Wirklichkeit aber wird eine andere Lösung dargestellt, die aus den Kurven 60b, 40b und 50b besteht, und in der Datenbank gespeichert war.

Das Verschieben kann durch Ergreifen mit einem Mauszeiger geschehen, oder durch unmittelbares Verschieben an einem Bildschirm eines Tablets.

Mit der Verschiebung des Schnittpunkts 61b, die eigentlich keine Aufwärtsverschiebung des Startpunktes im Navigationsabschnitt 76a ist, erfolgt real die Auswahl eines anderen Plans mit drei Dosis-Volumen-Graphen. Punkt 61a kann in diesem Abschnitt 76a zwischen dem oberen Endwert 75b und dem unteren, aufwärts verschobenen Schrankenwert 75d auf den Wert 75f verschoben werden. Eine dieser Verschiebungen ist in Figur 15 dargestellt und repräsentiert eine weitere Lösung mit den beispielhaften drei DVH-Graphen, die jetzt, statt der vorigen Lösung mit den drei Graphen 40a, 40b, 40c dargestellt wird sind Hilfsbilder 35, 36 und 37 auch vorhanden, werden auch diese aktualisiert. Im Beispiel ist das hauptsächlich das Hilfsbild 35. Mit der "Verschiebung" des Startwerts 61a zum neuen verschobenen Startwert 61b verschiebt (=verändert) sich auch der erste Startwert 41a im Steuerbereich 72, vgl. Figur 12, zu dem neuen, verschobenen Startwert 41b in Figur 15. Entsprechend wird auch der Startwert 51a der Kurve 50a "aufwärts verschoben", mit der Darstellung einer neuen Kurve 50b mit ihrem "verschobenen" Startwert 51b (auch als verschobener erster Startwert 51a zu 51a' symbolisiert).

Von den gespeicherten Lösungen können durch "Verschieben jedes Startwertes" auf jeder der geraden Achsen andere, weitere vorgespeicherte Lösungen dargestellt werden, die durch Darstellung entsprechender Kurventripel von beispielsweise 40c, 50c und 60c so erhalten werden.

Plausibel ist die Verschiebung der Dosis des Targets in Figur 15 so, dass eine Aufwärtsverschiebung eine höhere Belastung auch der anderen betrachteten Volumina R und N verursacht. Entsprechend werden alle Schnittpunkte aus Figur 14 aufwärts verschoben und liegen auf einem höheren Wert in Figur 15.

In Zahlen ist der Wert 61a von 92% auf 98% angehoben worden. Die anderen drei DVH-Kurven wurden dadurch vom System S aus Figur 1 aktualisiert dargestellt. Das Risikokriterium ist von 58% auf 71% erhöht worden, das übrige Körpergewebe auf Kurve 40b ist von 28% von Kurve 40a aus Figur 14 auf 32% in Kurve 40b in Figur 15 angehoben worden. Es soll betont werden, dass diese Anhebung keine Linearverschiebung ist, sondern durch Darstellung eines anderen, vorberechneten Plans in der Datenbank erhalten wird. Dieser neue dargestellte Plan hat die Werte 98%, 71% und 32% auf den Dosis-Graphen 60b, 50b und 40b.

Mit der Darstellung der **Figur 15a** ist symbolisch ein kleiner Abschnitt einer geraden, vertikal ausgerichteten Achse 70 gezeigt.

Links im Bild ist ein Originalbereich, der angenommen in einem Steuerbereich 76a nach Figur 15 liegen soll, aber oben und unten abgeschnitten dargestellt wird. Dieser Bereich, der innerhalb eines Steuerbereiches liegt, ist im rechten Halbbild der Figur 15a stark vergrößert in vertikaler Richtung dargestellt. Es sind "feste Stützstellen" aus den vorberechneten, gespeicherten Lösungen gegeben, diese werden a, $a_1$ und $a_2$ genannt. Diese festen Stützstellen sind Schnittpunkte von gleichwertigen Dosis-Volumen-Graphen, wie sie dem Graphen 60b von Figur 15 entsprechen, nur aus drei nicht gleichen Lösungen. Navigiert der Benutzer seinen Schnittpunkt (den Selektor) 61b nach aufwärts oder abwärts innerhalb des Steuerbereichs 76a, so kann er den Schnittpunkt a, den Schnittpunkt $a_1$ und den Schnittpunkt $a_2$ erreichen. Auf diesen Punkten gibt es eine Lösung, oder einen entsprechenden Dosis-Volumen-Graphen, der hier seinen Schnittpunkt mit der geraden Achse 70 (als Kriterium) besitzt.

Wirkungsmäßig verschiebt der Benutzer also seinen Selektor (oder den Schnittpunkt, der hier angenommen in "a" liegen soll), nach abwärts zu $a_1$ oder $a_2$. Tatsächlich, innerhalb des Systems S von Figur 1, werden neue Lösungen dargestellt, und hiervon jeweils nur eine.

Wenn dem Benutzer eine größere Kontinuität suggeriert werden soll, also seine Bewegung vom Punkt a ausgehend abwärts auch kleinere Züge erlauben soll, werden Zwischenlösungen berechnet. Jede der Zwischenlösungen hat einen Schnittpunkt mit der geraden Achse 70, die mit $d_1$, $d_2$... $d_{10}$ dargestellt sind. Diese Zwischenwerte liegen zwischen zwei oder noch mehreren schon vorhandenen Lösungen und werden von dem Berechnungsmodul 12 erstellt, das Zugriff zu den gespeicherten Lösungen der Datenbank 10 hat. Diese Zwischenlösungen werden über die Leitung 13a dem Visualisierungs-Modul 11 zugeführt, das diese über Leitung 11a anzeigen kann, abhängig von der Position, an welche der Benutzer den 'Selektor' auf dem geraden Abschnitt 70 von Figur 15a platziert.

Es wird dabei zu einem Zeitpunkt immer nur eine Lösung im DVH-Diagramm 30 dargestellt. "Verschiebt" der Benutzer seinen Selektor nach abwärts, wird eine nächste Lösung dargestellt, welche die dargestellte vorhergehende Lösung auf dem Dosis-Volumen-Histogramm 30 ablöst.

Es ist zu ersehen, dass die Abstände der vorberechneten Lösungen deutlich geringer werden und das Maß an Kontinuität erreicht wird, welches dem Benutzer das Gefühl gibt, er verschiebe einen Graphen im Dosis-Volumen-Histogramm. Die gefühlte Verschiebung selbst ist indes nur eine optische Wirkung, die dem Benutzer gegeben wird, vielmehr wählt er jeweils einen neuen Graphen aus, der parallel auch eine Änderung der anderen angezeigten Dosis-Volumen-Graphen mit sich bringt, sodass insgesamt ein neuer Plan dargestellt wird.

Aus einer beschränkten Anzahl von vorberechneten Lösungen werden durch die genannte Rekombination in der Berechnungseinheit 12 sehr viele Lösungen, sodass deren Schnittpunkte auf der Achse, die angewählt werden können, einen praktisch durchgehenden Steuerbereich bilden, wie das Figur 15a mit zehn Stützstellen und drei vorberechneten Werten in einem Abschnitt von 0,5 Volumenprozent zeigt.

Die folgenden **Figuren 15b, 15c und 15d** zeigen die Entstehung dieser Stützstellen durch eine rechnerische Rekombination.

In Figur 15b ist nur eine Lösung dargestellt, die im Beispiel - vereinfacht - drei Graphen im DVH-Diagramm 30 besitzt, wobei der mittlere Graph die vertikale, gerade Achse 70 schneidet und den Schnittpunkt b' definiert. Aus diesem Schnittpunkt heraus soll navigiert werden und um den Schnittpunkt herum gibt es dazu einen Steuerbereich (als Navigationsfenster), der in Figur 15c durch drei vorberechnete Pläne festgehalten ist. Dieser Navigations- oder Steuerbereich 70a hat drei Schnittpunkte, die obere Grenze, die untere Grenze und den Schnittpunkt b, der dem Plan b entspricht. Die anderen beiden Graphen haben zwar auch Schnittpunkte in Figur 15b, werden aber an dieser Stelle nicht betrachtet.

Es werden diejenigen Graphen in dem Steuerbereich 70a zusammengefasst, die einander entsprechen und als vorberechnete Lösungen in der Datenbank 10 vorhanden sind.

Wäre keine Zwischenberechnung eingesetzt, also keine Rekombination, stünden dem Benutzer nur drei anwählbare Punkte im Steuerbereich 70a zu Verfügung. Normalerweise sind es viel mehr Pläne, die vorberechnet zur Verfügung stehen, sodass ein Quasi-Kontinuum schon besteht, aber dieses Quasi-Kontinuum kann weiter dadurch verfeinert werden, dass eine Vielzahl von Stützstellen entsprechend der Darstellung der Figur 15a von Modul 12 berechnet werden, wie das mit Figur 15d symbolisiert ist.

Betrachtet man den beinahe-kontinuierlichen Bereich unter der Lupe, sieht man in Figur 15d, dass er sich aus vielen einzelnen Punkten zusammensetzt. Navigiert der Benutzer oder Planer mit seinem Selektor, "verschiebt" er also diesen Selektor auf der geraden Achse 70, so fühlt es sich für ihn an, dass der Wechsel von einer dargestellten Lösung zur nächsten dargestellten Lösung kontinuierlich ist. Er fühlt die Sprünge von einem Plan zum nächsten gar nicht mehr, weil die Pläne so dicht beieinanderliegen.

Die aus den gespeicherten Plänen a, b und c errechneten Zwischenpläne füllen beispielsweise den herausgegriffenen Bereich 70a' unterhalb vom Plan b auf, der einen Teilabschnitt des zuvor in Figur 15c beschriebenen Steuerbereichs 70a ist. In diesem Bereich 70a' fügt das Modul 12 aus Figur 1 die Vielzahl von Zwischenplänen ein, die unterhalb des Plans b liegen und ein gefühltes Kontinuum entstehen lassen, welches der Benutzer bei seiner Navigation gefühlsmäßig empfindet.

Eine solche Rekombination kann nach folgendem Muster berechnet werden.

Bei gewissen Übereinstimmungen der Einstellgrößen, z.B. bei Winkelpositionen und Energiewerten in den technischen Parametern eines Plan, sind vorhandene, vorberechnete Pläne miteinander "rekombinierbar", was einen Übergang von der ursprünglich diskreten Vielfalt solcher Pläne auf eine "kontinuierliche Vielfalt" erlaubt. Bezeichnen Vektoren $x_1$, $x_2$, ..., $x_N$ die Gesamtheit nichtnegativer Bestrahlungszeiten für die mit 1 bis N indizierten, miteinander rekombinierbaren Pläne, dann können diese über die gewichtete Summe ...

$$x_{komb} = w_1 \cdot x_1 + w_2 \cdot x_2 + ... + w_N \cdot x_N$$

mit den Koeffizienten $w_1$, $w_2$, ..., $w_N$ zu einer Gesamtheit $x_{komb}$ von nach Voraussetzung nichtnegativen Bestrahlungszeiten eines neuen Plans rekombiniert werden.

Zerfällt die Planvielfalt in mehrere Teilmengen jeweils miteinander rekombinierbarer Pläne, so erfolgt die Rekombination jeweils innerhalb dieser Teilmengen.

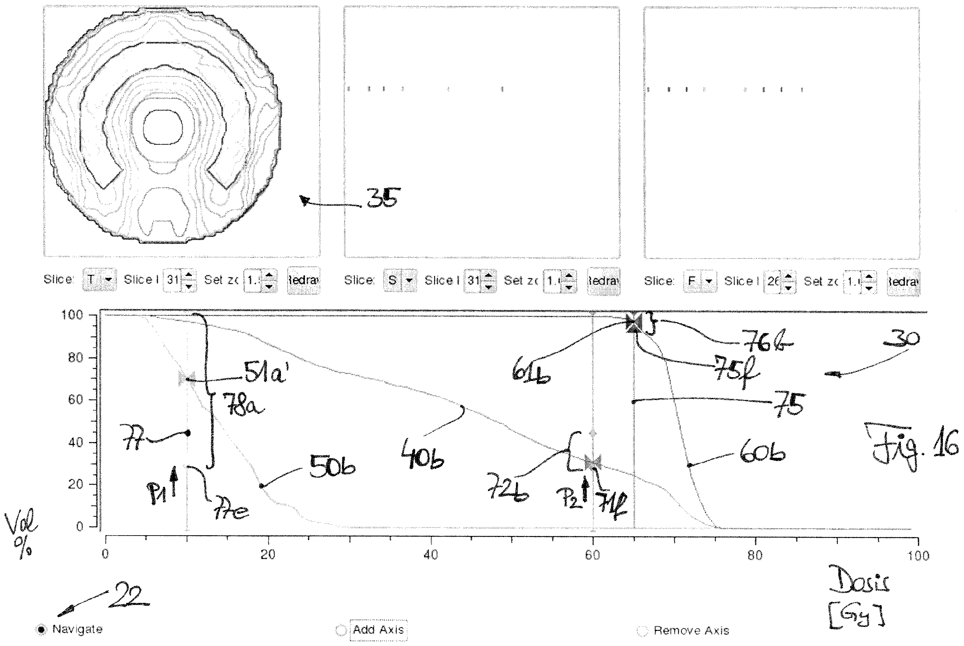

**Figur 16** festigt die gemäß Figur 15 erhaltene hohe Dosis auf dem Graphen 60b für den Tumor. Diese soll bei einem Wert von erreichten 98% bei der weiteren Navigation nicht wieder deutlich verschlechtert werden. Deshalb wird der untere Restriktor, der bislang 75d in Figuren 13/14 war, angehoben und auf eine neue Position von ca. 97% knapp unterhalb des verschobenen Startpunkts 61b platziert. Diese Platzierung hat eine Aktualisierung sämtlicher Steuerbereiche 72b, 78a zur Folge. Auf dem Graphen 60b für den Tumor (das Target) ist der neue, kleinere Steuerbereich 76b gegeben, der durch den aufwärts verschobenen unteren Restriktor 75f festgelegt wird. Damit kann sich der Schnittpunkt 61b im Steuerbereich 76b nicht weiter nach unten verschlechtern.

Durch die enge Vorgabe des Steuerbereichs 76b auf dem Risikographen 60b verbleibt für die anderen beiden geraden Achsen 71, 75 ein jeweils neuer Steuerbereich 72b und 78a, deren unterer Grenzwert 71f bzw. 77e ist. Beim Risiko ist der untere Wert auf 31% angehoben, statt der ursprünglichen 3% aus Figur 14, und für das übrige Gewebe auf dem Graphen 40b ist der untere Wert auf 31% angehoben statt der vorhergehenden 26% aus Figur 14. Die Pfeile P1, P2 zeigen die Richtung der Änderung.

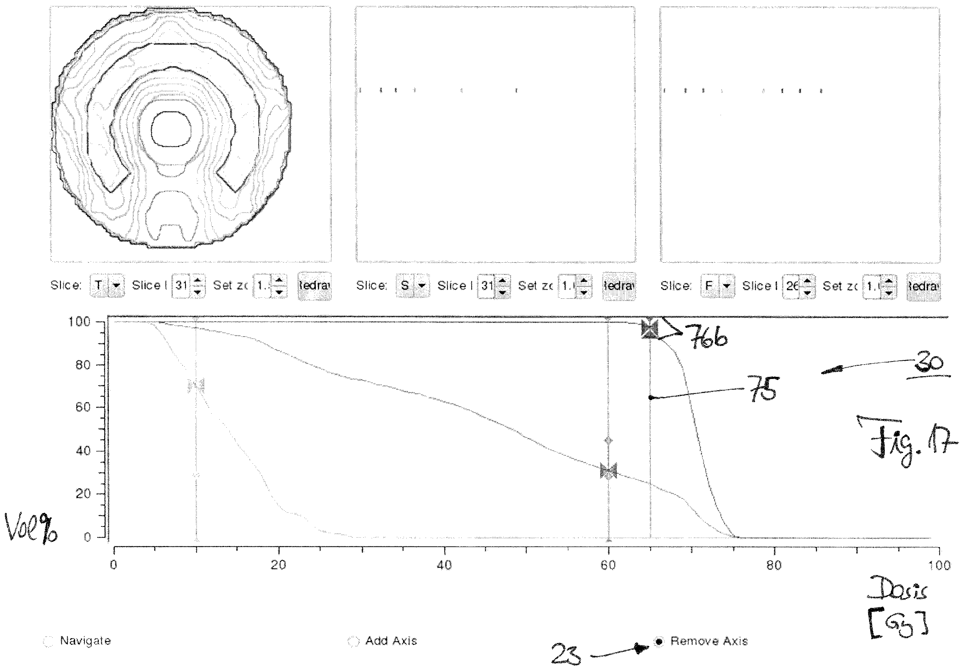

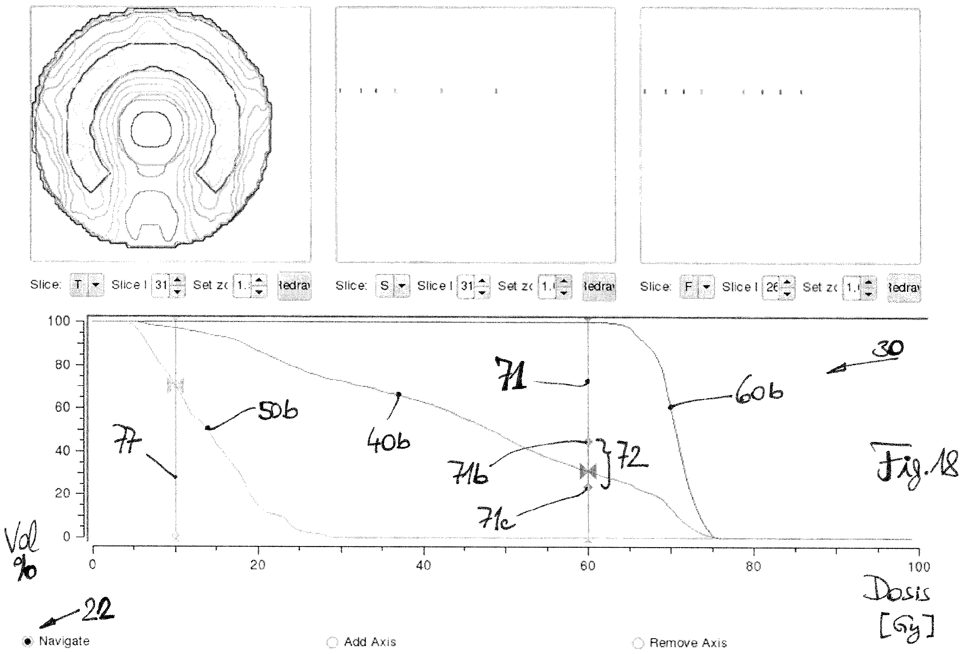

**Figur 17** veranschaulicht den Ausgangspunkt für das Entfernen einer zuvor festgelegten vertikalen Achse. Es soll die Tumorachse 75 entfernt werden. Der Modus-Auswahlselektor 23 wird aktiviert und durch Anklicken der Achse 75 wird diese entfernt, wie **Figur 18** zeigt.

Das Entfernen der geraden Achse 75 hat auch einen Einfluss auf die weitergehende Navigation, indem die Restriktion im engen Steuerbereich 76b aufgehoben wird, also mehr Lösungen für die anderen Steuerbereiche 72b und 78a freigegeben werden. Dadurch ergeben sich neue Steuerbereiche auf den noch vorhandenen geraden Achsen 71 und 77, die wieder die ursprüngliche Größe 72 für die gerade Achse 71 und 78 auf der geraden Achse 77 haben.

Nach Entfernen der vertikalen geraden Achse 75 (als 'Kriterium' für das Target) ergibt sich die Figur 18. Es ist noch immer die zweite Lösung eingezeichnet, welche die Graphen 40b, 50b und 60b besitzt. Durch die Modus-Auswahl (Button 21 auf "Navigation") kann in Figur 18 weiter navigiert werden, in den beschriebenen Arten der Begrenzung der Steuerbereiche oder der Platzierung von vertikalen geraden Achsen als Festlegung von Kriterien oder der "Verschiebung" der Schnittpunkte.

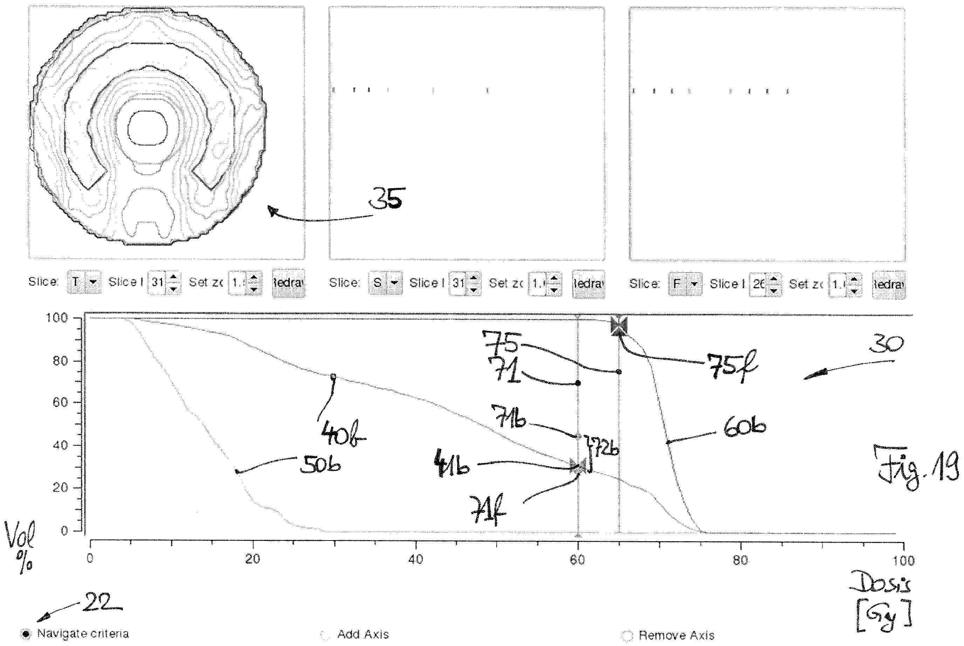

**Figur 19** zeigt den Beginn einer Extension. Die Extension ist eine Veränderung eines Steuerbereichs in einen noch nicht von vorberechneten Lösungen belegten Abschnitt auf der geraden Achse.

Diese soll anhand der **Figuren 19, 20, 21 und 22** erläutert werden.

Ausgangspunkt ist die Figur 19, die eigentlich der Figur 16 entspricht, ohne die dritte dort eingeblendete gerade Achse 77. Dargestellt ist die zweite und die erste eingeblendete gerade Achse 75 und 71 und der Schnittpunkt 41b auf der geraden Achse 71 liegt so, dass er nahe dem unteren Ende des Steuerbereichs 72b platziert ist. Dieser Schnittpunkt (Selektor) soll abwärts verschoben werden ("selektieren" neuer Lösungen). Das ist der Ausgangspunkt der Figur 19, deshalb ist als Modus "Navigation" (=Nav) eingeschaltet, mit dem Button 22.

Auf dem DVH-Graphen 40b im Diagramm 30 ist eine Abwärtsbewegung des Schnittpunktes 41b kaum noch möglich, es kann allenfalls der untere Endwert 71f des Steuerbereichs 72b angewählt werden und eine entsprechende Lösung statt der dargestellten Lösung 40b, 50b, 60b angezeigt werden. Mehr Spielraum ist für den Planer oder Benutzer nicht gegeben.

Die Extension hilft hier aus und erlaubt es die untere Grenze 71f weiter nach unten zu verlegen. Die untere Grenze 71f soll den Steuerbereich nach unten erweitern, wo es bislang keine vorberechneten Pläne in der Datenbank 10 gibt, die über das Visualisierungs-Modul 11 auf der Darstellungseinrichtung 20 im Bereich des DVH-Diagramms 30 dargestellt werden können.

Wissenschaftlich stellt sich das Ergebnis wie folgt dar. Die untere Restriktion im Tumorkriterium (Achse 77, Restriktor 75f in Figur 16) hatte zur Auswirkung, dass der Wertebereich im Kriterium des übrigen Körpergewebes (DVH-Graph 40b) knapp unterhalb der aktuellen Selektorposition endet, also eine Selektion zwecks Verbesserung in diesem Kriterium nicht möglich wäre. Deswegen wird beim Übergang zum nächsten Bild der untere Extender von 31 % (der Volumen-Prozentachse) nach unten verschoben, um den Wertebereich in dieser Richtung zu vergrößern.

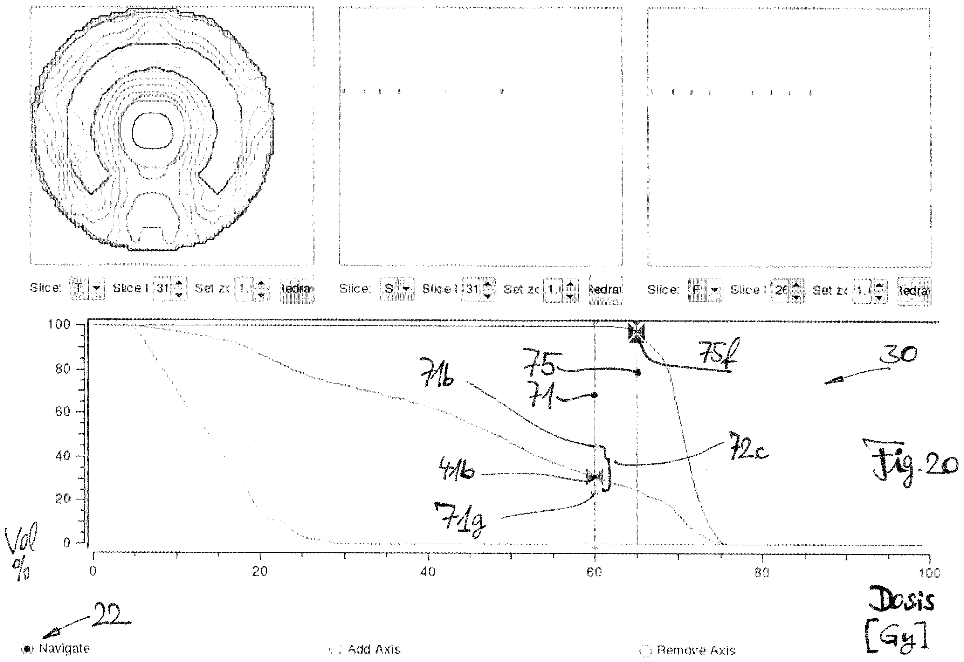

In der praktischen Anwendung erhält der Benutzer/Planer dann einen erweiterten Navigationsbereich, sein Steuerbereich 72c ist größer geworden in Figur 20, und zwar um den Linienabschnitt zwischen 41b und 71g.

Dieses Verlagern der unteren Grenze 71g von 71f aus Figur 19 ist zunächst ein Wunsch und eine Signalisierung im Sinne einer Steuerung (vom Tablet Schirm oder mit einem Mauszeiger), die über die bidirektionale Verbindung 11a dem Visualisierungs-Modul 11 übermittelt wird, und von dort wird eine Neuberechnung über das Berechnungsmodul 12 veranlasst, neue Pläne zu erzeugen und in der Datenbank 10 abzulegen.

Zunächst ist der Linienabschnitt zwischen 41b und 71g aber leer und kann von einer Veränderung oder Verlagerung des Schnittpunkts 41b auf der Achse 71 nicht angesteuert werden.

Nach Abschluss der Berechnungen vom Berechnungsmodul 12 liegen Zwischenpläne vor, die mit ihren Steuergrößen für das technische Gerät so ausgestaltet sind, dass sie die Lücke zwischen 41b und 71g auffüllen (als viele mögliche Schnittpunkte) und auch im übrigen Bereich einen vollständigen Graphen des DVH-Diagramms 30 bilden.

Die Hinzunahme der neuen Zwischenpläne zu den bestehenden, vorberechneten Bestandsplänen bewirkt eine Aktualisierung der verfügbaren Wertebereiche (der Steuerbereiche). Eine entsprechende Neupositionierung der 'Extender' in allen Kriterien (den geradlinigen Achsen, im Beispiel der Figur 20 die Achsen 71 und 75) erfolgt, falls erforderlich.

Ein Beispiel, wie die numerische Berechnung in dem Berechnungsmodul 12 vorgenommen werden kann, findet sich in **Philipp Süss**, A primal-dual barrier algorithm for the IMRT planning problem - An application to optimization-driven adaptive discretization, Dissertation, Fachbereich Mathematik, TU Kaiserslautern, mensch und buch verlag, Berlin (2008).

Das darin beschriebene Optimierungsverfahren geschieht in der Form, dass der/die resultierenden Kriterienwerte für das betrachtete Kriterium zwischen dem bisherigen Ende des verfügbaren Wertebereichs und dem gewünschten Kriterienwert liegen und in allen übrigen Kriterien innerhalb der durch die jeweiligen Restriktoren begrenzten Achsenabschnitte liegen.

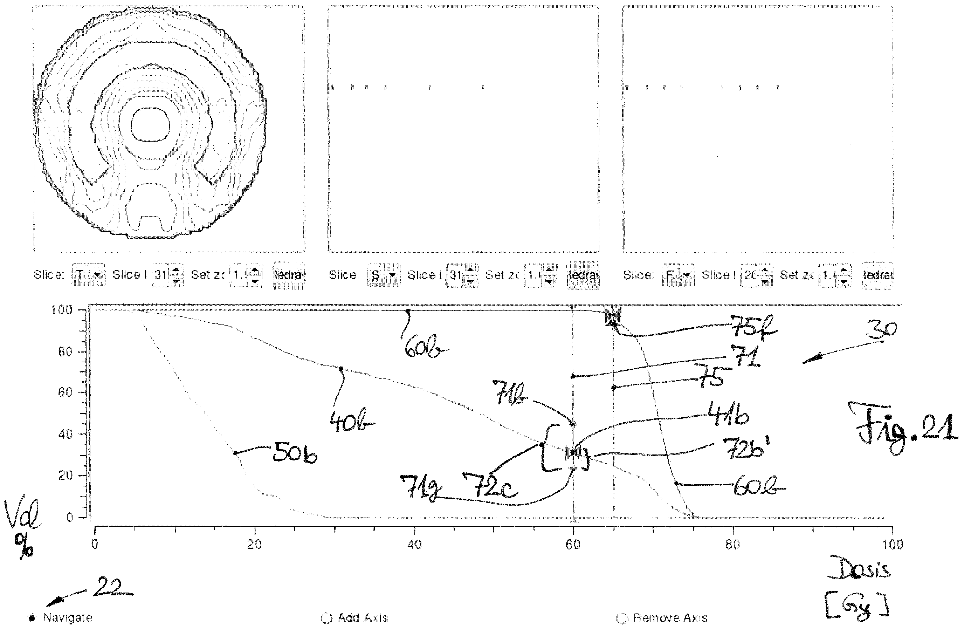

In Figur 20 ist der untere Rand des Steuerbereichs 72c verändert und beträgt jetzt nur noch 25% der Volumen-Prozentachse. Es ergibt sich das Bild der Figur 21, welches einen mit Schnittpunkten belegten Steuerbereichs 72c besitzt, in dem auch der Unterabschnitt 72b' oberhalb des unteren, neuen Randes 71g mit ansteuerbaren Werten für eine Veränderung des Schnittpunktes 41b nach unten zur Verfügung steht.

Die Dichte der nachberechneten Lösungen, die auch in der Datenbank 10 platziert sind, kann abhängig davon gemacht werden, wie groß oder lang der aufzufüllende Abschnitt 72b' relativ zum Ausgangsabschnitt 72b ist.

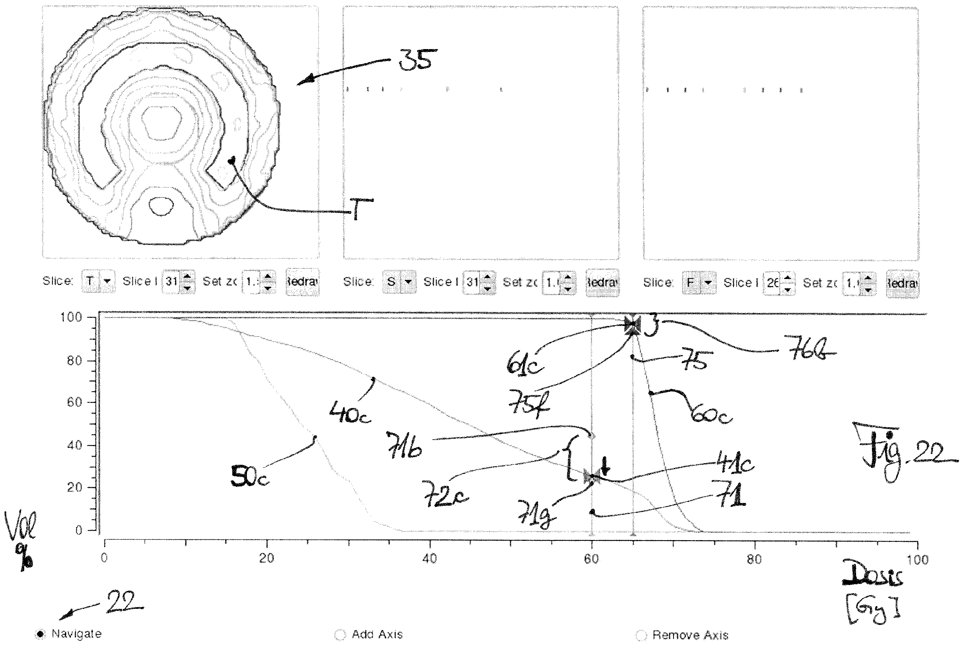

Im folgenden Bild der **Figur 22** ist das geschehen, wofür die Figuren 19 bis 21 die Vorbereitung getroffen haben. Das Berechnungsmodul 12 hat die Werte im Bereich 72b' aufgefüllt und der Selektor (der Schnittpunkt 41b) mit der geraden Achse 71 (dem Kriterium) ist nach abwärts verschoben worden, symbolisiert durch den kleinen Abwärtspfeil in Figur 22, und ein neuer Schnittpunkt 41c wählt einen der neuberechneten Graphen aus. Dieser Graph 40c gehört zu einer neuberechneten, dritten Lösung, die dargestellt wird und mit diesem Graphen werden auch die übrigen, zugehörigen Graphen, die hier die Graphen 60c und 50c im DVH-Diagramm 30 sind, aktualisiert. Mit der Aktualisierung der anderen Graphen wird auch das Hilfsbild 35 aktualisiert, welches die Isodosen darstellt.

Sichergestellt ist auch, dass der neuberechnete Graph 60c für das Tumorvolumen T innerhalb des vorgegebenen Steuerabschnitts 76b liegt, aber im abfallenden Ast deutlich steiler verläuft. Der Schnittpunkt 61c mit der Achse 75 des Steuerbereichs 76b (mit unterer Begrenzung 75f) kann sich verschieben, bleibt aber innerhalb des vorgegebenen Steuerbereichs 76b.

Qualitativ bewertet ist der mittlere Graph bei höheren Dosiswerten abwärts "verschoben worden", an sich sind durch das Abwärtsverschieben des Punktes 41b zum Punkt 41c andere Pläne dargestellt worden, die bei höheren Dosiswerten kleinere Volumen-Prozentwerte besitzen, dafür ist der Graph 50c für das Risiko in den Dosiswerten angestiegen und sein abfallender Ast endet nicht bei ca. 28 Gy, sondern bei ca. 37 Gy.

## Patentansprüche

1. **Interaktives Auswahlverfahren** des Auswählens einer Lösung aus einer Vielzahl von vorberechneten Lösungen, die in einer Datenbank (10) gespeichert vorliegen und auf einer Darstellungseinrichtung (20) dem auswählenden Benutzer interaktiv steuerbar (11) darstellfähig sind, wobei das interaktive Auswählen folgende Schritte umfasst;

   (a) Abbilden eines DVH-Diagramms (30), wobei DVH für ein Dosis-Volumen-Histogramm steht, mit mehreren DVH-Kurven (39;40a,50a,60a), das DVH-Diagramm als Hauptdiagramm (30) auf einem Bildschirm der Darstellungseinrichtung (20), wobei nur eine aus der Vielzahl von vorberechneten, gespeicherten Lösungen zu einem Zeitpunkt als Hauptdiagramm (30) visuell dargestellt wird;

   (b) Auswahl eines ersten Startpunkts (41a,61a) und Festlegen des Startpunkts auf einer ausgewählten (40a, 60a) der mehreren DVH-Kurven (39;40a, 50a,60a) im Hauptdiagramm (30),

   -- wodurch eine erste gerade Achse (71, 73, 75), die durch den ersten Startpunkt (41a,61a) verläuft, im Hauptdiagramm (30) ebenso dargestellt wird,

   -- wie ein optisch hervorgehobener, erster Steuerbereich (72,74,76), der um den ersten Startpunkt (41a, 61a) herum und auf der ersten geraden Achse (71,73,75) gelegen ist;

   -- zur Visualisierung der Vielzahl von in der Datenbank (10) gespeicherten, aktuell nicht als DVH-Kurven dargestellten Lösungen, deren, der ausgewählten DVH-Kurve (40a) entsprechende DVH-Kurven (40b,40c, ...) die erste gerade Achse (71,73) schneiden und dadurch eine Längserstreckung des ersten Steuerbereichs (72,74,76) festlegen;

   (c) wobei der erste Steuerbereich (72,74,76) einen oberen und einen unteren Endwert hat (71b,71c;73a,73b), die größter bzw. kleinster Wert der einander entsprechenden, gespeicherten DVH-Kurven sind, welche auch die erste gerade Achse (71,73,75) schneiden;

   (d) Abbilden zumindest eines weiteren Bedienelements (71e,75d) auf der ersten geraden Achse (71, 75), wobei

   -- der erste Startpunkt (41a,61a) ein zur Steuerung der Veränderung der ausgewählten DVH-Kurve (40a,60a) vom Benutzer interaktiv benutztes erstes Bedienelement ist

   -- und das weitere Bedienelement (71e, 75d) den ersten Steuerbereich (72, 74, 76) durch eine Verlagerung auf eine kleinere Längserstreckung (76a, 72a) einschränkt;

   (e) weitere Lösungen vom Benutzer interaktiv dadurch ausgewählt werden, dass der Startpunkt (41a) verlagert wird und durch seine Verlagerung (41a',41b) andere DVH-Kurven (40b,50b,60b) im Hauptdiagramm (30) von einem Visualisierungs-Modul (11) dargestellt werden, von denen eine dargestellte DVH-Kurve (40b) ebenso den ersten, eingeschränkten Steuerbereich (72) schneidet.

2. Verfahren nach Anspruch 1, wobei die erste gerade Achse (71,75) ein Achsenabschnitt ist, der länger als die Längserstreckung des zugehörigen Steuerbereichs (72,76) ist.

3. Verfahren nach Anspruch 1, wobei eine Lösung eine Gruppe von definierten Volumina (R,N,T) beinhaltet, wobei jedem Volumen der definierten Volumina eine visuell dargestellte DVH-Kurve (r,n,t) zugeordnet ist.

4. Verfahren nach Anspruch 1, wobei die gerade Achse die ausgewählte DVH-Kurve am Startpunkt schneidet, und der Startpunkt sowohl die Lage der geraden Achse (71,75) wie auch die ausgewählte DVH-Kurve (40a) definiert, insbesondere als gerade Achse auch ein gerader Abschnitt zu verstehen ist, der ausreichend lang ist.

5. Verfahren nach Anspruch 1, wobei drei Hilfsdiagramme (35,36,37) vorgesehen sind, welche drei Schnittdarstellungen in zueinander senkrechten Richtungen mit eingetragenen Isodosen sind, und - den mehreren DVH-Kurven (39) des Hauptdiagramms (30) entsprechende - Volumina in den Hilfsdiagrammen repräsentiert sind.

6. Verfahren nach Anspruch 1, wobei ein weiterer Startpunkt (61a) auf einer anderen der mehreren DVH-Kurven (39,40a,50a,60a) der ausgewählten Lösung festgelegt wird und eine zweite gerade Achse (75) durch den weiteren Startpunkt (61a) verlaufend dargestellt wird.

7. Verfahren nach einem der vorigen Ansprüche, wobei zumindest eine gerade Achse (71) vertikal verläuft.

8. Verfahren nach einem der vorigen Ansprüche, wobei zumindest eine Achse (73) der geraden Achsen (71,73) horizontal verläuft.

9. Verfahren nach einem der vorigen Ansprüche, wobei die erste gerade Achse (71) um einen Winkel im Bereich $\pm 89°$ verdrehbar ist, dies um ihren Startpunkt (71a*,41a) herum.

10. Verfahren nach einem der vorigen Ansprüche, wobei Zwischenlösungen aus gespeicherten, vorberechneten Lösungen der Datenbank (10) berechnet werden (12) und für eine Verlagerung des Startpunkts (41a) als neue Schnittpunkte $(d_1,d_2,...)$ in dem ersten Steuerbereich (72) verfügbar sind.

11. Verfahren nach einem der vorigen Ansprüche, wobei der erste Steuerbereich (72b) um einen Streckenabschnitt (72b') erweitert wird, mit einer Berechnung (12) von Lösungen, zugehörigen DVH-Kurven (40c,50c,60c) im Hauptdiagramm (30) und außerhalb seiner anfänglichen Erstreckung auf seiner geraden Achse (71) liegender Schnittpunkte (41c) mit derselben geraden Achse (71), und der möglichen Darstellung dieser Schnittpunkte, wobei jeder zu einer berechneten Lösung gehörige Schnittpunkt (41c) und alle zu derselben Lösung gehörigen DVH-Kurven (40c,50c,60c) im Hauptdiagramm (30) dargestellt werden, wenn der zugehörige Schnittpunkt (41c) im erweiterten Streckenabschnitt (72b') ausgewählt wird.

12. Verfahren nach einem der vorigen Ansprüche, wobei zumindest eine Hilfsdarstellung (35,36,37) abgebildet wird, welche die aktuell in dem Hauptdiagramm (30) abgebildete Lösung (40a,50a,60a) auf eine andere Weise visualisiert.

13. Verfahren nach einem der vorigen Ansprüche, wobei mehrere Bedienelemente auf der zumindest einen geraden Achse (71,75) zur Interaktion dargestellt sind, ein Selektor als Start- oder Schnittpunkt (41a) und/oder Restriktoren (71e,75d) zur Begrenzung eines oberen und unteren Grenzwerts des ersten Steuerbereichs (72,76) auf einen kürzeren Steuerbereich (72a,76a).

14. Verfahren nach Anspruch 13, wobei der Restriktor (71e, 75d) nicht über den aktuellen Schnittpunkt hinausbewegbar ist und damit der Start- oder Schnittpunkt (41a) immer zwingend im ersten Steuerbereich (72,76) liegt.

15. Verfahren nach einem der vorigen Ansprüche, wobei Zwischenlösungen aus vorhandenen, vorberechneten Lösungen der Datenbank (10) berechnet werden (12), und der erste Steuerbereich (72) aus den Schnittpunkten mit der Achse (71) ergänzt und auf dem Hauptdiagramm (30) dargestellt wird.

16. Verfahren nach einem der vorigen Ansprüche, wobei während einer jeweiligen Verlagerung des Startpunktes (41a, 41a') eine jeweilige Zwischenlösung aus vorhandenen, vorberechneten Lösungen der Datenbank (10) berechnet wird (12) und in dem Hauptdiagramm (30) jeweils so anzeigbar ist, dass der Schnittpunkt einer jeweiligen DVH Kurve (40b) der Zwischenlösung die gerade Achse (71) am verlagerten Startpunkt (41a') schneidet.

17. Verfahren nach einem der vorigen Ansprüche, wobei das weitere Bedienelement (71e,75d) durch seine Verlagerung den ersten Steuerbereich (72,76) auf eine kleinere Längserstreckung (72a,76a) von oben oder von unten bei einer vertikalen ersten geraden Achse (71,75) reduziert oder einschränkt.

**18.** Verfahren nach Anspruch 13 oder 15, wobei die Verlagerung des weiteren Bedienelementes (71e;75d) zur Einschränkung des ersten Steuerbereichs (72;76) und damit Ausschluss von Lösungen zur Aktualisierung der ersten Steuerbereiche auf allen geraden Achsen (72,76) mit einer Entfernung der Schnittpunkte der ausgeschlossenen Lösungen führt.

**19.** Verfahren nach Anspruch 6, wobei ein weiterer Steuerbereich (76) um den zweiten Startpunkt (61a) herum auf der zweiten geraden Achse (75) optisch hervorgehoben auf der Darstellungseinrichtung (20) dargestellt wird.

**20.** Verfahren nach Anspruch 1 oder 19, wobei mehrere gerade Achsen (71,75,77) durch mehrere Schnittpunkte mit zwei oder drei Kurven (40a,50a,60a) verlaufen, welche geraden Achsen bevorzugt parallel orientiert sind.

**Claims**

**1. Interactive selection method** of selecting a solution from a plurality of precalculated solutions stored in a database (10) and displayable (11) on a display device (20) in an interactively controllable manner to the selecting user, wherein the interactive selection comprises the following steps;

(a) imaging a DVH diagram (30), wherein DVH stands for a dose-volume histogram, with a plurality of DVH curves (39;40a,50a,60a), the DVH diagram as a main diagram (30) on a screen of display means (20), wherein only one of the plurality of precalculated stored solutions at a time is visually displayed as the main diagram (30);
(b) selecting a first starting point (41a, 61a) and setting the starting point on a selected one (40a, 60a) of the plurality of DVH-curves (39; 40a, 50a, 60a) in the main diagram (30),

-- whereby a first straight axis (71, 73, 75) passing through the first starting point (41a, 61a) is also shown in the main diagram (30),
-- like a visually highlighted first control area (72,74,76) located around the first starting point (41a, 61a) and on the first straight axis (71,73,75);
-- for visualizing the plurality of solutions stored in the database (10) and currently not represented as DVH curves, whose DVH curves (40b, 40c, ...) corresponding to the selected DVH curve (40a) intersect the first straight axis (71, 73) and thereby define a longitudinal extension of the first control region (72, 74, 76);

(c) wherein said first control section (72,74,76) has upper and lower end values (71b,71c;73a,73b) which are respectively the largest and smallest values of the corresponding stored DVH curves which also intersect said first straight axis (71,73,75);
(d) imaging at least one further control element (71e, 75d) on the first straight axis (71, 75), wherein

-- the first starting point (41a, 61a) is a first control element used interactively by the user to control the change of the selected DVH-curve (40a, 60a)
-- and the further control element (71e, 75d) restricts the first control range (72, 74, 76) by shifting to a smaller longitudinal extent (76a, 72a);

(e) further solutions are interactively selected by the user by shifting the starting point (41a) and by its displacement (41a', 41b) other DVH curves (40b, 50b, 60b) are displayed in the main diagram (30) by a visualization module (11), of which a displayed DVH curve (40b) also intersects the first, restricted control area (72).

**2.** Method according to claim 1, wherein the first straight axis (71,75) is a portion of the axis which is longer than the longitudinal extent of the associated control region (72,76)

**3.** Method according to claim 1, wherein a solution comprises a group of defined volumes (R,N,T), wherein a visually represented DVH curve (r,n,t) is associated with each volume of the defined volumes.

**4.** Method according to claim 1, wherein the straight axis intersects the selected DVH curve at the starting point, and the starting point defines both the position of the straight axis (71, 75) and the selected DVH curve (40a), in particular a straight axis also being a straight section which is sufficiently long.

**5.** Method according to claim 1, wherein three auxiliary diagrams (35, 36, 37) are provided, which are three sectional representations in mutually perpendicular directions with isodoses entered, and -volumes corresponding to -the several DVH curves (39) of the main diagram (30) -are represented in the auxiliary diagrams.

6. Method according to claim 1, wherein a further starting point (61a) is set on another one of a plurality of DVH curves (39, 40a, 50a, 60a) of the selected solution and a second straight axis (75) is represented passing through the further starting point (61a).

7. Method according to one of the previous claims, where at least one straight axis (71) is vertical.

8. Method according to one of the previous claims, whereby at least one axis (73) of the straight axes (71,73) is horizontal.

9. Method according to one of the previous claims, whereby the first straight axis (71) is rotatable through an angle in the range ±89°, this around its starting point (71a*,41a).

10. Method according to one of the previous claims, wherein intermediate solutions are calculated (12) from stored, precalculated solutions of the database (10) and are available for a displacement of the starting point (41a) as new intersection points (d1,d2,...) in the first control range (72)

11. Method according to one of the previous claims, wherein the first control range (72b) is extended by a section of line (72b'), with a calculation (12) of solutions, associated DVH curves (40c,50c,60c) in the main diagram (30) and points of intersection (41c) with the same straight axis (71) lying outside its initial extension on its straight axis (71), and the possible representation of these intersection points, wherein each intersection point (41c) belonging to a calculated solution and all DVH curves (40c,50c,60c) belonging to the same solution are represented in the main diagram (30) when the associated intersection point (41c) is selected in the extended line section (72b').

12. Method according to one of the previous claims, wherein at least one auxiliary representation (35, 36, 37) is depicted, which visualizes the solution (40a, 50a, 60a) currently depicted in the main diagram (30) in a different way.

13. Method according to one of the previous claims, wherein a plurality of control elements are represented on the at least one straight axis (71, 75) for interaction, a selector as starting point or intersection (41a) and/or restrictors (71e, 75d) for limiting an upper and lower limit value of the first control range (72, 76) to a shorter control range (72a, 76a).

14. Method according to claim 13, wherein the restrictor (71e, 75d) cannot be moved beyond the current intersection point and thus the starting or intersection point (41a) is always necessarily located in the first control range (72, 76).

15. Method according to one of the previous claims, whereby intermediate solutions are calculated (12) from existing, precalculated solutions of the database (10), and the first control range (72) is supplemented from the intersections with the axis (71) and displayed on the main diagram (30).

16. Method according to one of the previous claims, wherein during a respective displacement of the starting point (41a, 41a') a respective intermediate solution is calculated (12) from existing, precalculated solutions of the database (10) and can be displayed in the main diagram (30) in each case in such a way that the point of intersection of a respective DVH curve (40b) of the intermediate solution intersects the straight axis (71) at the displaced starting point (41a').

17. Method according to one of the previous claims, wherein the further control element (71e,75d) by its displacement reduces or restricts the first control area (72,76) to a smaller longitudinal extension (72a,76a) from above or from below at a vertical first straight axis (71,75).

18. Method according to claim 13 or 15, wherein the displacement of the further control element (71e;75d) leads to the restriction of the first control range (72;76) and thus exclusion of solutions for updating the first control ranges on all straight axes (72,76) with a distance of the intersections of the excluded solutions.

19. Method according to claim 6, wherein a further control area (76) around the second starting point (61a) on the second straight axis (75) is displayed optically highlighted on the display device (20).

20. Method according to claim 1 or 19, wherein a plurality of straight axes (71,75,77) pass through a plurality of intersections with two or three curves (40a,50a,60a), which straight axes are preferably oriented parallel.

**Revendications**

1. **Procédé de sélection interactive** pour sélectionner une solution parmi une pluralité de solutions précalculées stockées dans une base de données (10) et affichables (11) sur un dispositif d'affichage (20) d'une manière contrôlable interactivement pour l'utilisateur qui fait la sélection, dans lequel la sélection interactive comprend les étapes suivantes ;

   (a) l'imagerie d'un diagramme DVH (30), où DVH signifie un histogramme dose-volume, avec une pluralité de courbes DVH (39 ; 40a, 50a, 60a), le diagramme DVH comme diagramme principal (30) sur un écran de moyens d'affichage (20), où une seule de la pluralité de solutions stockées précalculées à la fois est affichée visuellement comme diagramme principal (30) ;
   (b) sélectionner un premier point de départ (41a, 61a) et fixer le point de départ sur une courbe sélectionnée (40a, 60a) de la pluralité de-courbes DVH (39 ; 40a, 50a, 60a) dans le diagramme principal (30),

   -- où un premier axe droit (71, 73, 75) passant par le premier point de départ (41a, 61a) est également représenté dans le diagramme principal (30);
   -- comme une première zone de contrôle mise en évidence visuellement (72,74,76) située autour du premier point de départ (41a, 61a) et sur le premier axe droit (71,73,75) ;
   -- pour visualiser la pluralité de solutions stockées dans la base de données (10) et actuellement non représentées sous forme de courbes DVH, dont les courbes DVH (40b, 40c, ...) correspondant à la courbe DVH sélectionnée (40a) coupent le premier axe droit (71, 73) et définissent ainsi une extension longitudinale de la première région de contrôle (72, 74, 76) ;

   (c) dans lequel ladite première section de contrôle (72, 74, 76) a des valeurs d'extrémité supérieure et inférieure (71b, 71c ; 73a, 73b) qui sont respectivement les valeurs les plus grandes et les plus petites des courbes DVH stockées correspondantes qui coupent également ledit premier axe droit (71, 73, 75) ;
   (d) l'imagerie d'au moins un autre élément de contrôle (71e, 75d) sur le premier axe droit (71, 75), dans laquelle

   -- le premier point de départ (41a, 61a) est un premier élément de contrôle utilisé de manière interactive par l'utilisateur pour contrôler le changement de la courbe DVH-sélectionnée (40a, 60a)
   -- et l'élément de contrôle supplémentaire (71e, 75d) restreint la première plage de contrôle (72, 74, 76) en se déplaçant vers une étendue longitudinale plus petite (76a, 72a) ;

   (e) d'autres solutions sont sélectionnées de manière interactive par l'utilisateur en déplaçant le point de départ (41a) et par son déplacement (41a', 41b) ; d'autres courbes DVH (40b, 50b, 60b) sont affichées dans le diagramme principal (30) par un module de visualisation (11), dont une courbe DVH affichée (40b) coupe également la première zone de contrôle restreinte (72).

2. Procédé selon la revendication 1, dans laquelle le premier axe droit (71,75) est une partie de l'axe qui est plus longue que l'étendue longitudinale de la région de contrôle associée (72,76).

3. Procédé selon la revendication 1, dans lequel une solution comprend un groupe de volumes définis (R,N,T), dans lequel une courbe DVH représentée visuellement (r,n,t) est associée à chaque volume des volumes définis.

4. Procédé selon la revendication 1, dans lequel l'axe droit coupe la courbe DVH sélectionnée au point de départ, et le point de départ définit à la fois la position de l'axe droit (71, 75) et la courbe DVH sélectionnée (40a), en particulier un axe droit étant également une section droite qui est suffisamment longue.

5. Procédé selon la revendication 1, dans laquelle trois diagrammes auxiliaires (35, 36, 37) sont fournis, qui sont trois représentations en coupe dans des directions mutuellement perpendiculaires avec des isodoses entrées, et les -volumes correspondant aux -plusieurs courbes DVH (39) du diagramme principal (30) -sont représentés dans les diagrammes auxiliaires.

6. Procédé selon la revendication 1, dans lequel un point de départ supplémentaire (61a) est fixé sur une autre courbe parmi une pluralité de courbes DVH (39, 40a, 50a, 60a) de la solution sélectionnée et un deuxième axe droit (75) est représenté passant par le point de départ supplémentaire (61a).

7. Procédé selon l'une des revendications précédentes, où au moins un axe droit (71) est vertical.

8. Procédé selon l'une des revendications précédentes, selon laquelle au moins un des axes (73) des axes droits (71,73) est horizontal.

9. Procédé selon l'une des revendications précédentes, selon laquelle le premier axe droit (71) peut tourner d'un angle de l'ordre de $\pm 89°$, ceci autour de son point de départ (71a*,41a).

10. Procédé selon l'une des revendications précédentes, dans laquelle des solutions intermédiaires sont calculées (12) à partir de solutions stockées et précalculées de la base de données (10) et sont disponibles pour un déplacement du point de départ (41a) comme nouveaux points d'intersection (d1,d2,...) dans la première plage de contrôle (72).

11. Procédé selon l'une des revendications précédentes, dans laquelle la première plage de contrôle (72b) est prolongée par une section de ligne (72b'), avec un calcul (12) des solutions, des courbes DVH associées (40c, 50c, 60c) dans le diagramme principal (30) et des points d'intersection (41c) avec le même axe droit (71) se trouvant en dehors de son extension initiale sur son axe droit (71), et la représentation possible de ces points d'intersection, où chaque point d'intersection (41c) appartenant à une solution calculée et toutes les courbes DVH (40c, 50c, 60c) appartenant à la même solution sont représentées dans le diagramme principal (30) lorsque le point d'intersection associé (41c) est sélectionné dans la section de ligne étendue (72b').

12. Procédé selon l'une des revendications précédentes, dans laquelle au moins une représentation auxiliaire (35, 36, 37) est représentée, qui visualise la solution (40a, 50a, 60a) actuellement représentée dans le diagramme principal (30) d'une manière différente.

13. Procédé selon l'une des revendications précédentes, dans lequel une pluralité d'éléments de contrôle sont représentés sur au moins un axe droit (71, 75) pour l'interaction, un sélecteur comme point de départ ou intersection (41a) et/ou des limiteurs (71e, 75d) pour limiter une valeur limite supérieure et inférieure de la première plage de contrôle (72, 76) à une plage de contrôle plus courte (72a, 76a).

14. Procédé selon la revendication 13, dans laquelle le limiteur (71e, 75d) ne peut pas être déplacé au-delà du point d'intersection actuel et donc le point de départ ou d'intersection (41a) est toujours nécessairement situé dans la première plage de contrôle (72, 76).

15. Procédé selon l'une des revendications précédentes, selon laquelle des solutions intermédiaires sont calculées (12) à partir de solutions existantes et précalculées de la base de données (10), et la première plage de contrôle (72) est complétée à partir des intersections avec l'axe (71) et affichée sur le diagramme principal (30).

16. Procédé selon l'une des revendications précédentes, dans lequel, lors d'un déplacement respectif du point de départ (41a, 41a'), une solution intermédiaire respective est calculée (12) à partir de solutions existantes, précalculées de la base de données (10) et peut être représentée dans le diagramme principal (30) dans chaque cas de telle sorte que le point d'intersection d'une courbe DVH respective (40b) de la solution intermédiaire coupe l'axe droit (71) au point de départ déplacé (41a').

17. Procédé selon l'une des revendications précédentes, dans lequel l'élément de contrôle supplémentaire (71e, 75d), par son déplacement, réduit ou restreint la première zone de contrôle (72, 76) à une plus petite extension longitudinale (72a, 76a) par le haut ou par le bas à un premier axe vertical droit (71, 75).

18. Procédé selon la revendication 13 ou 15, dans laquelle le déplacement de l'élément de contrôle supplémentaire (71e ; 75d) conduit à la restriction de la première plage de contrôle (72 ; 76) et donc à l'exclusion des solutions pour la mise à jour des premières plages de contrôle sur tous les axes droits (72, 76) avec une distance des intersections des solutions exclues.

19. Procédé selon la revendication 6, dans lequel une autre zone de contrôle (76) autour du deuxième point de départ (61a) sur le deuxième axe droit (75) est affichée en surbrillance optique sur le dispositif d'affichage (20).

20. Procédé selon la revendication 1 ou 19, dans lequel une pluralité d'axes droits (71, 75, 77) passent par une pluralité d'intersections avec deux ou trois courbes (40a, 50a, 60a), lesquels axes droits sont de préférence orientés parallèlement.

Figur 1

EP 2 795 496 B1

Fig. 3

35

36

37

Slice: T ▾  Slice I 31 ▴▾  Set z⟨ 1.⟨ ▴▾  ?edra\

Slice: S ▾  Slice I 31 ▴▾  Set z⟨ 1.⟨ ▴▾  ?edra\

Slice: F ▾  Slice I 2⟨ ▴▾  Set z⟨ 1.⟨ ▴▾  ?edra\

R  T      N

100
80
60
40
20
0

Vol %

n

t

30

r

Fig. 4

0        20        40        60        80        100

Dosis [Gy]

○ Navigate        ● Add Axis        ○ Remove Axis

Slice: T ▾   Slice I 31 ↕   Set z( 1. ↕   Redra

Slice: S ▾   Slice I 31 ↕   Set z( 1. ↕   Redra

Slice: F ▾   Slice I 2( ↕   Set z( 1. ↕   Redra

35

36a

37a

R   N   T

30

100
80
60
40
20
0

r   h   t   41a

Vol %

0   20   40   60   80   100

Dosis [Gy]

Fig.5

○ Navigate      ● Add Axis      ○ Remove Axis

EP 2 795 496 B1

N

T

R

35

Slice: T ▾  Slice I 31 ⬍  Set z( 1. ⬍  Redra    Slice: S ▾  Slice I 31 ⬍  Set z( 1. ⬍  Redra    Slice: F ▾  Slice I 26 ⬍  Set z( 1. ⬍  Redra

60a

30

40

Fig.6

40a

71b

71

50a

41a

[71a]

72

71c

Vol
%

22

Dosis
[Gy]

• Navigate            ○ Add Axis            ○ Remove Axis

Fig. 8

Fig.9

Slice: T ▾  Slice I 31 ▴▾  Set z( 1.: ▴▾  Redra

Slice: S ▾  Slice I 31 ▴▾  Set z 1. ▴▾  Redra

Slice: F ▾  Slice I 2( ▴▾  Set z( 1. ▴▾  Redra

35

30

40a

41a

73c

73

73

73a  74  73b

73d

Vol %

Dosis [Gy]

100
80
60
40
20
0

0    20    40    60    80    100

● Navigate        ○ Add Axis        ○ Remove Axis

22

EP 2 795 496 B1

Fig. 10

Fig. 11

EP 2 795 496 B1

Fig.12

EP 2 795 496 B1

31

Fig. 13

Slice: T ▾  Slice I 31 ⬍  Set z( 1. ⬍  Redra    Slice: S ▾  Slice I 31 ⬍  Set z( 1. ⬍  Redra    Slice: F ▾  Slice I 26 ⬍  Set z( 1. ⬍  Redra

71
75
71e
75d
76a
30
40a
60a
72a

Vol %

Dosis [Gy]

● Navigate          ○ Add Axis          ○ Remove Axis

33

Slice: T ▾ Slice I 31 ▴▾ Set zc 1.5 ▴▾ Redraw    Slice: S ▾ Slice I 31 ▴▾ Set zc 1.! ▴▾ Redraw    Slice: F ▾ Slice I 26 ▴▾ Set zc 1.! ▴▾ Redraw

Vol %

22

● Navigate            ○ Add Axis            ○ Remove Axis

77d
78
51a
77
77c
50a
40a
72
71
75
61a
76a
75d
30

Fig.14

Dosis [Gy]

Slice: [T ▾] Slice I [31 ▴▾] Set zo [1. ▴▾] Redra    Slice: [S ▾] Slice I [31 ▴▾] Set zo [1. ▴▾] Redra    Slice: [F ▾] Slice I [26 ▴▾] Set zo [1. ▴▾] Redra

Fig. 15

● Navigate                    ○ Add Axis                    ○ Remove Axis

EP 2 795 496 B1

Figur 15a

Plan b

3 Kurven

Figur 15b

Figur 15c

Vol. [%]

70

b'

Plan b

Dosis [Gy]

Vol. [%]

70

a

b

c

70a

Dosis [Gy]

a

b

c

Berechnung erfolgt mit
Berechnungsmodul 12
aus Fig. 1

Figur 15d

b

a

c

Vol. [%]

70

a

b

c

70a

70a'

Dosis [Gy]

70a'

b

70a'

Fig. 16

Slice: T ▾ Slice I 31 ⬍ Set z⟨ 1. ⬍ Redra

Slice: S ▾ Slice I 31 ⬍ Set z⟨ 1. ⬍ Redra

Slice: F ▾ Slice I 26 ⬍ Set z⟨ 1. ⬍ Redra

Vol%

Dosis [%]

766
75
30

Fig. 17

23

○ Navigate          ○ Add Axis          ● Remove Axis

Fig.18

Fig. 13

Fig. 20

Fig. 21

Fig. 22

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7391026 B2
- WO 2011153639 A
- US 20050111621 A

**In der Beschreibung aufgeführte Nicht-Patentliteratur**

- **TU KAISERSLAUTERN.** Dissertation, Fachbereich Mathematik. mensch und buch verlag, 2008